(19) Europäisches Patentamt
European Patent Office
Office européen des brevets

(11) Veröffentlichungsnummer: **0 044 932**
**B1**

(12) **EUROPÄISCHE PATENTSCHRIFT**

(45) Veröffentlichungstag der Patentschrift: **03.04.85**

(21) Anmeldenummer: **81104569.9**

(22) Anmeldetag: **13.06.81**

(51) Int. Cl.[4]: **C 07 D 493/04** // C07D473/06 (C07D493/04, 307:00, 307:00)

(54) **Gegebenenfalls N-substituierte Amino-desoxy-1.4;3.6-dianhydro-hexit-Derivate, Verfahren zu ihrer Herstellung und deren Verwendung.**

(30) Priorität: **25.07.80 DE 3028288**

(43) Veröffentlichungstag der Anmeldung: **03.02.82 Patentblatt 82/05**

(45) Bekanntmachung des Hinweises auf die Patenterteilung: **03.04.85 Patentblatt 85/14**

(84) Benannte Vertragsstaaten: **AT BE CH FR GB IT LI NL SE**

(56) Entgegenhaltungen:
**US-A-3 886 186**

**Chemical Abstracts Band 89, Nr. 5, 31. Juli 1978 Colmbus, Ohio, USA V.P. ARYA "Derivatives of isosorbide" Seite 612, Spalte 1, Abstract Nr. 43188x**
**Chemical Abstracts Band 44, Nr. 12, 25. Juni 1950 Columbus, Ohio, USA V.G. BASHFORD et al. "Anhydrides of polyhydric alcohols. XIII. The amino derivatives of 1,4:3,6-dianhydromannitol,-sorbitol, and L-iditol and their behavior towards nitrous acid" Abstract Nr. 5314g**

(73) Patentinhaber: **Dr. Willmar Schwabe GmbH & Co.**
**Willmar-Schwabe-Strasse 4**
**D-7500 Karlsruhe 41 (DE)**

(72) Erfinder: **Klessing, Klaus, Dr., Dipl.-Chem.**
**Rheingoldstrasse 3**
**D-7505 Ettlingen 5 (DE)**

(74) Vertreter: **Bunke, Holger, Dr. Dipl.-Chem. et al**
**Patentanwälte Prinz, Bunke & Partner**
**Lessingstrasse 9**
**D-7000 Stuttgart 1 (DE)**

(56) References cited:
**Chemical Abstracts Band 44, Nr. 3, 10. Februar 1950 Columbus, Ohio, USA V.G. BASHFORD et al. "Interconversion of dianhydro hexitols and of saccharic acids" Abstract Nr. 1024d**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 044 932 B1

**Beschreibung**

Die Erfindung betrifft Amino-desoxy-1.4;3.6-dianhydrohexit-Derivate der allgemeinen Formel I,

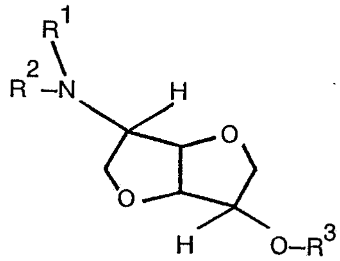

(I)

worin $R^1$ und $R^2$ jeweils unabhängig voneinander ein Wasserstoffatom oder eine Niedrigalkylgruppe mit 1 bis 4 C-Atomen

oder worin $R^1$ ein Wasserstoffatom und $R^2$ einen Adamant(1)yl-Rest

oder worin $R^1$ und $R^2$ gemeinsam mit dem Stickstoffatom, an das sie gebunden sind, (a) den Rest eines cyclischen, nicht-aromatischen, gegebenenfalls ein weiteres Heteroatom enthaltenden sekundären Amins, das ausgewählt ist aus der Gruppe Pyrrolidin, Piperidin, Morpholin, Methylpiperazin, oder (b) den Aden(9)yl-Rest, den 6-Alkylamino-purin(9)yl- oder 6-Dialkylamino-purin(9)yl-Rest, wobei die Alkylgruppen 1 bis 4 C-Atome aufweisen, den gegebenenfalls halogensubstituierten 6-(ω-Phenylalkyl)-amino-purin(9)yl-Rest, dessen Alkylgruppe 1 bis 8 C-Atome aufweist, oder den 6-Pyrrolidinopurin(9)yl-, 6-Piperidinopurin(9)yl-, 6-Morpholinopurin(9)yl- oder 6-Methylpiperazinopurin(9)yl-Rest, oder (c) den 6-Alkylmercaptopurin(9)yl-Rest oder (d) den Theophyllin(7)yl-Rest oder (e) den 6-Chlorpurin(9)yl-Rest

oder worin $R^1$ ein Wasserstoffatom oder eine Niedrigalkylgruppe mit 1 bis 4 C-Atomen und $R^2$ einen ω-Theophyllin(7)yl-alkyl-Rest oder einen ω-Theobromin-1-yl-alkyl-Rest oder einen ω-(N,N'-diniedrigalkyl-substituierten Xanthin-N''-yl)-alkyl-Rest, wobei "niedrigalkyl" eine Alkylgruppe mit 1 bis 5 C-Atomen bedeutet, oder einen ω-Adenin-9-yl-alkyl-Rest, wobei der Alkylrest jeweils 2 bis 7 C-Atome aufweisen und geradkettig oder verzweigt sein kann,

und worin $R^3$ ein Wasserstoffatom, eine Methansulfonyl- oder Toluolsulfonylgruppe bedeuten, sowie deren Säureadditionssalze.

Das Grundgerüst dieser Verbindungen besteht aus einem der stereoisomeren, unter Epimerisierung ineinander umwandelbaren 1.4;3.6-Dianhydro-hexite, nämlich entweder dem 1.4;3.6-Dianhydro-L-idit (="Isoidid") (II),

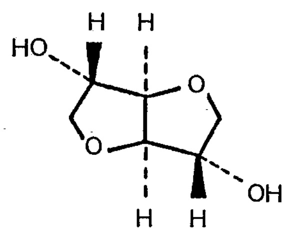

(II)

bei dem die OH-Gruppen in 2- und 5-Stellung jeweils exo-Konfiguration aufweisen, oder dem 1.4;3.6-Dianhydro-D-glucit (="Isosorbid") (III),

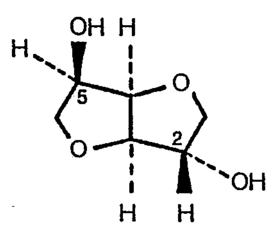

(III)

der eine exo-ständige und eine endo-ständige OH-Gruppe aufweist und somit—bei verschiedenen Substituenten in 2- und 5-Stellung—in zwei isomeren Formen auftritt.

Des Grundgerüst einiger Verbindungen schließlich besteht aus dem 1.4;3.6-Dianhydro-D-mannit (="Isomannid") (IV),

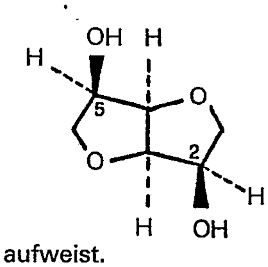

(IV)

der zwei endo-ständige OH-Gruppen aufweist.

Da im Gegensatz zu den Glucit-Derivaten bei den Idit- und Mannit-Derivaten eine Unterscheidung zwischen den 2- und 5-Substituenten nicht möglich ist, weil das $C^2$-Atom bei Drehung des Moleküls um 180° zum $C^5$-Atom wird, sind Hinweise auf die 5-Stellung oder 2-Stellung von Substituenten bei diesen Derivaten an sich überflüssig. Des besseren Vergleiches der Strukturen der einzelnen Verbindungen mit den allgemeinen Formeln wegen werden aber hier die Isoidid-Derivate durchweg als 5-Amino-isoidid-Derivate bezeichnet, da sie aus den in 5-Stellung acylsubstituierten Isosorbid-Derivaten entstehen. Entsprechend werden die als Ausgangsverbindungen verwendeten Isomannid-Acyl-Derivate als 2-Acylisomannid-Derivate bezeichnet, da aus ihnen die 2-Amino-isosorbid-Derivate hergestellt werden.

Eine kurze Zusammenfassung über die Stereoisomerie der 1.4;3.6-Dianhydro-hexite gibt J. A. Mills in Advances in Carbohydrate Chem. *10,* 1—53 (1955).

Die erfindungsgemäßen Verbindungen sind wertvolle Zwischenprodukte zur Herstellung der in EP—A—44 927, 44 928 und 44 940 beschriebenen gegebenenfalls N-substituierten Amino-desoxy-1.4;3.6-dianhydro-hexit-mononitrate der allgemeinen Formel (Ia),

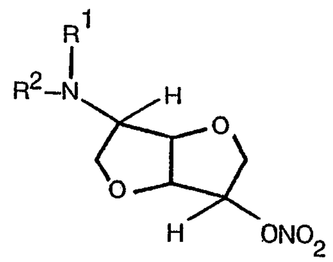

(Ia)

worin $R^1$ und $R^2$ die vorstehend genannten Bedeutungen besitzen.

Die Verbindungen der allgemeinen Formel (Ia) besitzen koronardurchflußsteigernde, spasmolytische, blutdrucksenkende, negativ inotrope und die Herzfrequenz senkende Wirksamkeit. Sie sind zur Behandlung von Koronarerkrankungen, zur Kupierung und Prophylaxe von Angina-pectoris-Anfällen, zur Nachbehandlung von Herzinfarkten und zur Behandlung von Herzinsuffizienzen geeignet. Sie bewirken darüber hinaus eine Verbesserung der peripheren Durchblutung und der Gehirndurchblutung.

Da sich gezeigt hatte, daß die beispielsweise aus der US—PS—3 886 186 bekannten 2- und 5-Mononitrate sowie das 2,5-Dinitrat des Isosorbids, die ebenfalls pharmakologisch wirksame Substanzen mit hämodynamischer, vasodilatorischer und antianginöser Wirksamkeit sind, unangenehme Nebenwirkungen, insbesondere Kopfschmerzen, verursachen, teilweise schlecht resorbierbar und teilweise—da es sich bei den Dinitraten um Explosivstoffe handelt—nur unter besonderen Vorsichtsmaßnahmen herstellen und handhaben lassen, bestand ein Bedürfnis nach der Bereitstellung neuer pharmazeutischer Mittel mit gleichem Wirkungsspektrum, die die Nachteile der bekannten Isosorbidmononitrate (ISMN) und des Isosorbiddinitrats (ISDN) nicht aufweisen, sowie nach der Schaffung neuer 1.4;3.6-Dianhydro-hexit-mononitrate, die als wirksame Bestandteile solcher pharmazeutischer Mittel verwendet werden können.

Die der Erfindung zugrundeliegende Aufgabe besteht darin, das angeführte Bedürfnis zu befriedigen, die Lösung dieser Aufgabe darin, die erfindungsgemäßen Verbindungen als reaktive Zwischenprodukte zur Herstellung der neuen 1.4;3.6-Dianhydro-hexit-mononitrate bereitzustellen.

Gegenstand der Erfindung sind demnach

1. 5-Amino-5-desoxy-1.4;3.6-dianhydro-L-idit-Derivate der allgemeinen Formel V,

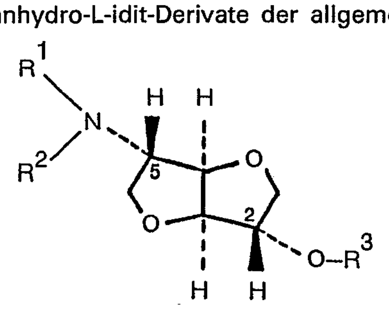

(V)

worin $R^1$, $R^2$ und $R^3$ die in Anspruch 1 genannten Bedeutungen besitzen, sowie deren Säureadditionssalze;

2. 5-Amino-5-desoxy-1.4;3.6-dianhydro-D-glucit-Derivate der allgemeinen Formel VI

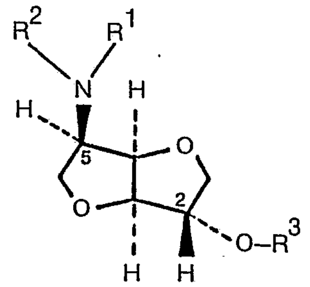

(VI)

worin $R^1$, $R^2$ und $R^3$ die in Anspruch 1 genannten Bedeutungen besitzen, sowie deren Säureadditionssalze;

3. 2-Amino-2-desoxy-1.4;3.6-dianhydro-D-glucit-Derivate der allgemeinen Formel VII,

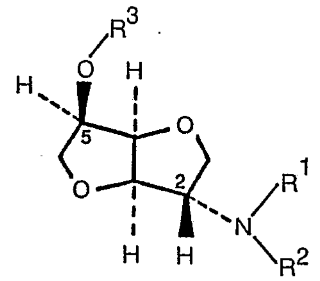

(VII)

worin $R^1$, $R^2$ und $R^3$ die in Anspruch 1 genannten Bedeutungen besitzen, sowie deren Säureadditionssalze;

4. 2-Amino-2-desoxy-1.4;3.6-dianhydro-D-mannit-Derivate der allgemeinen Formel VIII,

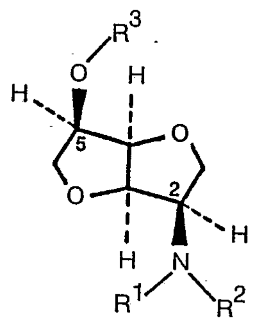

(VIII)

worin $R^1$, $R^2$ und $R^3$ die in Anspruch 1 genannten Bedeutungen besitzen, sowie deren Säureadditionssalze;

5. (Purin-9-yl)-desoxy-1.4;3.6-dianhydro-hexit-Derivate der allgemeinen Formel IX,

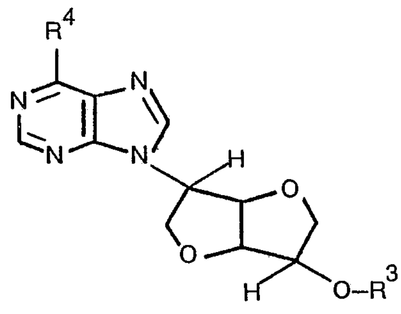

(IX)

worin $R^3$ ein Wasserstoffatom, eine Methansulfonyl- oder Toluolsulfonylgruppe und

$R^4$ eine Aminogruppe, eine Alkylmercapto-, Alkylamino- oder Dialkylaminogruppe mit 1 bis 4 C-Atomen, eine ω-Phenylalkylaminogruppe, deren Alkylrest 1 bis 8 C-Atome aufweist und deren Phenylrest gegebenenfalls halogensubstituiert ist, oder den Rest eines cyclischen, nicht-aromatischen, gegebenenfalls ein weiteres Heteroatom enthaltenden sekundaren Amins das ausgewählt ist aus der Gruppe Pyrrolidin, Piperidin, Morpholin, Methylpiperazin, oder ein Chloratom bedeuten, sowie deren Säureadditionssalze;

6. 5-(Purin-9-yl)-5-desoxy-1.4;3.6-dianhydro-L-idit-Derivate der allgemeinen Formel X,

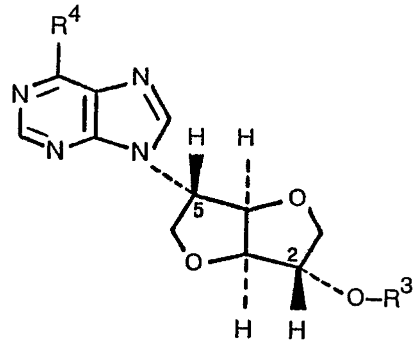

(X)

worin $R^3$ und $R^4$ die in Anspruch 6 genannten Bedeutungen besitzen, sowie deren Säureadditionssalze;

7. 5-(Purin-9-yl)-5-desoxy-1.4;3.6-dianhydro-D-glucit-Derivate der allgemeinen Formel XI,

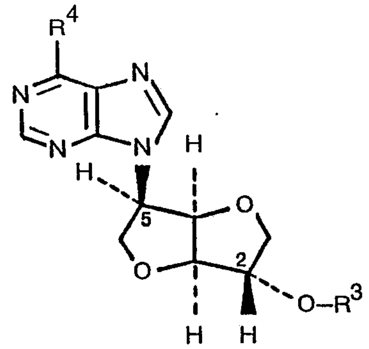

(XI)

worin $R^3$ und $R^4$ die in Anspruch 6 genannten Bedeutungen besitzen, sowie deren Säureadditionssalze;

8. 2-(Purin-9-yl)-2-desoxy-1.4;3.6-dianhydro-D-glucit-Derivate der allgemeinen Formel XII,

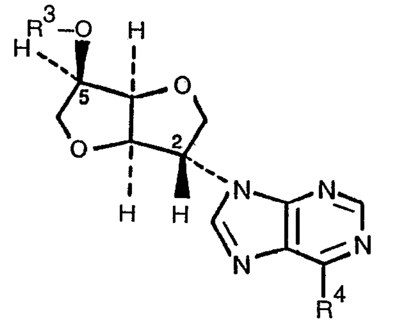

(XII)

worin $R^3$ und $R^4$ die in Anspruch 6 genannten Bedeutungen besitzen, sowie deren Säureadditionssalze;

9. 2-(Purin-9-yl)-2-desoxy-1.4;3.6-dianhydro-D-mannit-Derivate der allgemeinen Formel XIII,

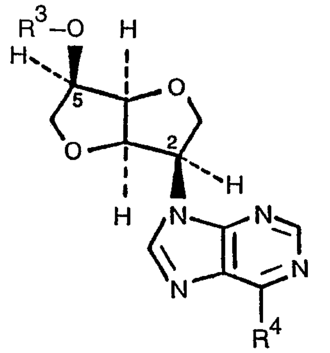

(XIII)

worin $R^3$ und $R^4$ die in Anspruch 6 genannten Bedeutungen besitzen, sowie deren Säureadditionssalze;

10. (Theophyllin-7-yl)-desoxy-1.4;3.6-dianhydro-hexit-Derivate der allgemeinen Formel XIV,

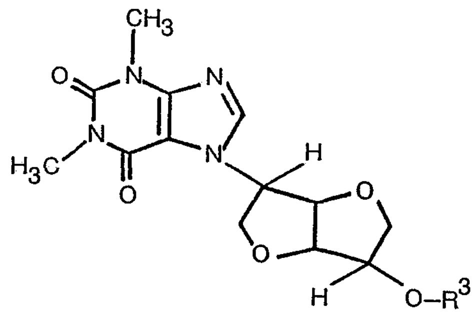

(XIV)

worin $R^3$ ein Wasserstoffatom, eine Methansulfonyl- oder Toluolsulfonylgruppe bedeutet, sowie deren Säureadditionssalze;

11. (ω-Purinylalkylamino)-desoxy-1.4;3.6-dianhydro-hexit-Derivate der allgemeinen Formel XV,

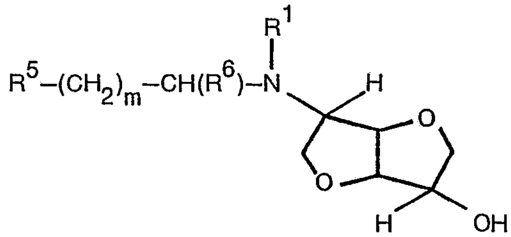

(XV)

worin $R^1$ ein Wasserstoffatom oder eine Niedrigalkylgruppe mit 1 bis 4 C-Atomen,

$R^5$ den Adenin-9-yl-Rest, den Theophyllin-7-yl-Rest, den Theobromin-1-yl-Rest oder einen weiteren N,N'-diniedrigalkylxanthin-N''-yl-Rest, wobei "niedrigalkyl" eine Alkylgruppe mit 1 bis 5 C-Atomen bedeutet,

$R^6$ ein Wasserstoffatom oder eine Methylgruppe und m eine ganze Zahl von 1 bis 6 bedeuten, sowie deren Säureadditionssalze;

12. ω-(Theophyllin-7-yl)-alkylamino-desoxy-1.4;3.6-dianhydro-hexite der allgemeinen Formel XVI,

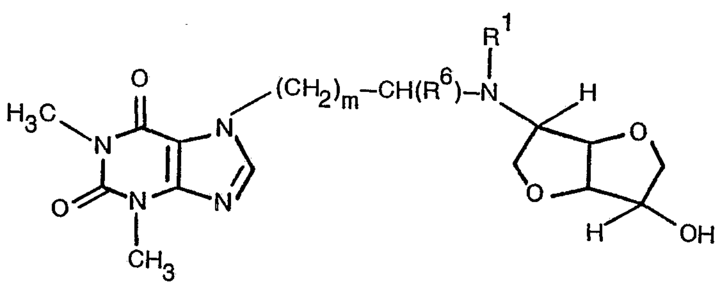

(XVI)

worin $R^1$, $R^6$ und m die in Anspruch 12 genannten Bedeutungen besitzen, sowie deren Säureadditionssalze;

13. verschiedene Verfahren zur Herstellung der erfindungsgemäßen Verbindungen und

14. die Verwendung der erfindungsgemäßen Verbindungen als reaktive Zwischenprodukte zur Herstellung der entsprechenden Amino-desoxy-1.4;3.6-dianhydro-hexit-nitrate.

Wie aus den vorstehenden allgemeinen Formeln hervorgeht, werden unter "Amino"-Derivaten auch solche Derivate verstanden, bei denen das Stickstoffatom Bestandteil eines aromatischen oder nicht-aromatischen heterocyclischen Ringsystems ist und das gegebenenfalls mit einem weiteren heterocyclischen Ringsystem anelliert ist, insbesondere auch Purin-Derivate wie gegebenenfalls 6-N-substituierte Adenin-, Theophyllin- oder andere Dialkylxanthin-Derivate.

Die erfindungsgemäßen Verbindungen besitzen im 1.4;3.6-Dianhydro-hexit-Grundgerüst vier asymmetrische C-Atome und liegen in optisch aktiver Form vor, da als Ausgangsverbindungen optisch reine 1.4;3.6-Dianhydro-hexite verwendet werden, die aus natürlich vorkommenden Zuckern oder Zuckeralkoholen leicht zugänglich sind.

Die Weiterverarbeitung der erfindungsgemäßen reaktiven Zwischenprodukte zu den Endprodukten, also den entspechenden Mononitraten, erfolgt so, daß die freie Hydroxylgruppe des entsprechenden, gegebenenfalls N-substituierten Amino-desoxy-1.4;3.6-dianhydro-hexits ($R^3$=H), die gegebenenfalls vorher durch alkalische Hydrolyse des entsprechenden Mesylats oder Tosylats ($R^3$=Methansulfonyl-,

Toluolsulfonyl-) geschaffen wird, mit Salpetersäure, Nitriersäure oder mit einem Gemisch aus Salpetersäure und Eisessig/Acetanhydrid, gegebenenfalls in Gegenwart von Harnstoff zum entsprechenden Amino-desoxy-1.4;3.6-dianhydro-hexit-nitrat verestert wird. Die Weiterverarbeitung ist auch in den bereits genannten drei gleichzeitig eingereichten Patentanmeldungen beschrieben (vgl. insbesondere die dortigen Beispiele).

Die erfindungsgemäßen Verbindungen können ausgehend von den epimeren, unsubstituierten 1.4;3.6-Dianhydro-hexiten, also ausgehend von L-Isoidid, D-Isosorbid und D-Isomannid, hergestellt werden, wobei jeweils mehrere verschiedene Synthesewege möglich sind.

Einer dieser Wege besteht erfindungsgemäß darin, daß der entsprechende 1.4;3.6-Dianhydro-hexit mit einem Sulfonsäurechlorid, vorzugsweise mit Methansulfonsäurechlorid oder Toluolsulonsäurechlorid, in einem geeigneten wasserfreien Lösungsmittel und in Gegenwart einer Hilfsbase, vorzugsweise in Pyridin oder in Chloroform/Triäthylamin, bei erniedrigter Temperatur, vorzugsweise zwischen −20° und +10°C, in den entsprechenden Mono - O - acyl - 1.4;3.6 - dianhydro - hexit übergeführt wird, welcher dann durch Zusatz einer wäßrigen, beispielsweise 25%igen Ammoniaklösung oder durch Zusatz eines primären oder sekundären Alkylamins mit 1 bis 4 C-Atomen, durch Zusatz von 1-Aminoadamantan oder durch Zusatz eines cyclischen, nicht-aromatischen, gegebenenfalls ein weiteres Heteroatom enthaltenden sekundären Amins, gegebenenfalls unter Zusatz eines geeigneten Lösungsmittels, einer Aminolyse unterworfen wird, und zwar vorteilhaft unter erhöhtem Druck, vorzugsweise bei einem Druck von 2 bis 20 at, und erhöhter Temperatur, vorzugsweise bei 90 bis 180°C. Die Aminolyse wird zweckmäßig in einem geschlossenen Stahlautoklaven bis zur quantitativen Umsetzung druchgeführt. Geeignete Lösungsmittel sind—gegebenenfalls unter Zusatz von Wasser—z.B. Alkohole, Di- oder Polyglykoyläther oder Dioxan.

Die Aminolyse des entsprechenden Mono-O-acyl-1.4;3.6-dianhydro-hexits kann auch unter Normaldruck, jedoch ebenfalls bei erhöhter Temperatur durchgeführt werden, nämlich dann, wenn anstelle eines primären oder sekundären Amins ein Alkalisalz des Adenins, 6-Chlor- oder 6-Alkylmercaptopurins oder des Theophyllins zugesetzt wird, wobei man vorzugsweise dipolare aprotische Lösungsmittel, z.B. Dimethylformamid oder Dimethylsulfoxid, zusetzt und vorzugsweise bei Temperaturen zwischen 90 und 120°C arbeitet und das Reaktionsgemisch mehrere Tage lang rührt.

Bei der Aminolyse zum entsprechenden, gegebenenfalls N-mono- oder -di-substituierten Amino - desoxy - 1.4;3.6 - dianhydro - hexit wird die Mesylat- bzw. Tosylatgruppe durch die Amino- oder substituierte Aminogruppe bzw. durch den gegebenenfalls substituierten Purinylrest nach dem Reaktionsmuster einer typischen bimolekularen nucleophilen Substitution ($S_N2$-Reaktion) ausgetauscht, die stets mit einer Umkehr der Konfiguration am zentralen Kohlenstoffatom verbunden ist. Diese Konfigurationsumkehr, dem Fachmann auch unter den Begriffen "Inversion" oder "Walden-Umkehr" geläufig, ist der Grund dafür, daß aus dem 1.4;3.6 - Dianhydro - D - glucit - 5 - acyl - Derivat, bei dem der Acylrest in 5-Stellung endo-ständig vorliegt, stets das in 5-Stellung durch die Aminogruppe oder eine entsprechend substituierte Aminogruppe oder den gegebenenfalls substituierten Purinylrest substituierte 1.4;3.6 - Dianhydro - L - idit - Derivat entsteht, bei dem der anstelle des Acylrests in das Molekül eingetretene Substituent nicht mehr in endo-, sondern in exo-Stellung steht. Die mit der $S_N2$-Reaktion verbundene Walden-Umkehr ist in ganz entsprechender Weise verantwortlich dafür, daß aus dem entsprechenden Idit-acylat stets das in 5-Stellung endo-substituierte Glucit-Derivat, aus dem Mannit-acylat das entsprechende, in 2-Stellung exo-substituierte Glucit-Derivat und aus dem Glucit-2-exo-acylat das entsprechende, in 2-Stellung endo-substituierte Mannit-Derivat entsteht.

Zur Herstellung derjenigen erfindungsgemäßen Verbindungen der allgemeinen Formel I, worin $R^1$ und $R^2$ gemeinsam mit dem Stickstoffatom, an das sie gebunden sind, einen an der 6-Aminogruppe mono- oder disubstituierten Aden(9)yl-Rest bedeuten, wird zunächst, wie vorstehend beschrieben, der entsprechende 6-Chlorpurin-9-yl- oder 6 - Alkylmercaptopurin - 9 - yl - desoxy - 1.4;3.6 - dianhydro - hexit hergestellt, und zwar durch Umsetzung des 6-Chlorpurin- oder 6 - Alkymercaptopurin - 9 - natriumsalzes mit dem entsprechenden Monoacyldesoxy - 1.4;3.6 - dianhydro - hexit. Der 6-Chlorpurin-9-yl- oder 6-Alkylmercaptopurin - 9 - yl - desoxy - 1.4;3.6 - dianhydro - hexit wird dann bei einer Temperatur zwischen 90 und 150°C im geschlossenen Autoklaven mit einer wäßrigen Alkylamin- oder Dialkylamin-Lösung, wobei die Alkylgruppe 1 bis 4 C-Atome aufweist, mit einem cyclischen, nicht-aromatischen, gegebenenfalls ein weiteres Heteroatom enthaltenden sekundären Amin das ausgewählt ist aus der Gruppe Pyrrolidin, Piperidin, Morpholin, Methylpiperazin, oder mit einem gegebenenfalls halogensubstituierten ω-Phenylalkylamin, dessen Alkylgruppe 1 bis 8 C-Atome aufweist, umgesetzt, wobei gegebenenfalls Wasser oder ein niederer Alkohol als Lösungsmittel zugesetzt wird.

Beispiele für die hierfür eingesetzten primären und sekundären Amine sind: Methylamin, Äthylamin, Propylamin, Isopropylamin, Butylamin, Dimethylamin, Diäthylamin, Dipropylamin, Dibutylamin, Pyrrolidin, Piperidin, Morpholin, Methylpiperazin, Benzylamin, 2-Phenyl-äthylamin, 3-Phenylpropylamin, 4-Phenyl-butylamin, 5-Phenyl-pentylamin, 6-Phenyl-hexylamin, 7-Phenyl-heptylamin, 8-Phenyl-octylamin sowie die entsprechenden im Phenylring ortho-, meta- oder para-halogensubstituierten ω-Phenyl-alkylamine.

Zur Herstellung derjenigen erfindungsgemäßen Verbindungen, bei denen das Stickstoffatom des Amino - desoxy - isohexid - Ringsystem durch eine geradkettige, unsubstituierte Alkylgruppe mit einem der Ring-Stickstoffatome eines Purinderivats, also eines Adenin-, Xanthin-, Theophyllin- oder Theobromin-Derivats, verbunden ist, wird der entsprechende Amino - desoxy - 1.4;3.6 - dianhydro - hexit

vorzugsweise mit einem N-(ω-Brom-, ω-Chlor- oder ω - Methansulfonyloxy - alkyl) - theophyllin, -theobromin, -dialkylxanthin oder -adenin umgesetzt, vorzugsweise mit 7 - (2 - Bromäthyl) - theophyllin, 7 - (3 - Chlorpropyl) - theophyllin, 7 - (3 - Brompropyl) - theophyllin, 7 - (3 - Methansulfonyloxypropyl) - theophyllin, 7 - (4 - Brombutyl) - theophyllin, 7 - (5 - Brompentyl) - theophyllin, 7 - (6 - Bromhexyl) - theophyllin und dergleichen.

Zur Vermeidung einer Disubstitution am Stickstoffatom der Aminogruppe des Aminoisohexids kann diese Aminogruppe vor der Umsetzung mit dem reaktiven Purinderivat mit einer als Schlutzgruppe geeigneten Verbindung, beispielsweise einer Benzylverbindung, umgesetzt werden, die sich nach der Umsetzung leicht wieder abspalten läßt, beispielsweise durch Hydrierung in Gegenwart eines üblichen Edelmetallkatlysators.

Ein weiteres geeignetes Verfahren zur Herstellung derjenigen erfindungsgemäßen Verbindungen, bei denen die "Brücke" zwischen dem Aminoisohexid und dem Purinderivat eine Alkylengruppe ist, besteht darin, daß man den entsprechenden Aminoisohexid zusammen mit einem ω-Acyl-alkyl- bzw. ω-Oxoalkyl-xanthin- oder -adenin-Derivat in an sich bekannter Weise einer reduktiven Kondensation in Gegenwart von Wasserstoff und einem geeigneten Edelmetall- oder Edelmetallsulfid-Katalysator, gegebenenfalls in Gegenwart eines Lösungsmittels, unterwirft. Im Falle der ω-Oxoalkyl-xanthin- oder -adenin-Derivate, beispielsweise des 7 - (3 - Oxopropyl) - theophyllins, entstehen die entsprechenden Purinyl-alkylamino-isohexid-Derivate mit unverzweigter Alkylenbrücke, beispielsweise 3 - Theophyllin - 7 - yl - propylamino - isohexid. Im Falle der ω-Acylalkyl-xanthin- oder -adenin-Derivate, z.B. des 7 - (2 - Acetyläthyl) - theophyllins oder des 7-(4-Acetylbutyl)-theophyllins, entstehen bei der reduktiven Kondensation mit dem Aminoisohexid die entsprechenden ω-Purinyl-alkylamino-iso-hexid-Derivate mit verzweigter Alkylengruppe, beispielsweise 3 - Theophyllin - 7 - yl - 1 - methyl - propylamino - isohexid oder 5 - Theophyllin - 7 - yl - 1 - methyl - pentylamino - isohexid. Die so erhaltenen erfindungsgemäßen Verbindungen mit verzweigter Alkylenbrücke besitzen ein zusätzliches Asymmetriezentrum, so daß jeweils zwei Diastereomere bei der reduktiven Kondensation entstehen, welche mit Hilfe üblicher Trennmethoden, beispielsweise durch Säulenchromatographie, Flüssig-Flüssig-Verteilung oder fraktionierte Kristallisation in die beiden Isomeren aufgetrennt werden können, also beispielsweise in (+) - 5 - (3 - Theophyllin - 7 - yl - 1 - methyl - propylamino) - 5 - desoxy - 1.4;3.6 - dianhydro - L - idit und (−) - 5 - (3 - Theophyllin - 7 - yl - 1 - methyl - propylamino) - 5 - desoxy - 1.4;3.6 - dianhydro - L - idit. Die Auftrennung kann auch erst nach Weiterverarbeitung zu den Salpetersäureestern stattfinden. Der bisher beschriebene erste erfindungsgemäße Weg zur Herstellung der erfindungsgemäßen Verbindungen, bei dem der entsprechende Isohexid mit einem Sulfonsäurechlorid, vorzugsweise mit Methansulfonsäurechlorid oder Toluolsulfonsäurechlorid, in den entsprechenden Monoacyl - 1.4;3.6 - dianhydro - hexit übergeführt wird, hat den Nachteil, daß bei der Acylierung nicht nur das entsprechende 5 - O - Acyl - Derivat bzw. 2-O-Acyl-Derivat entsteht, sondern gleichzeitig auch das 2,5-Diacyl-Derivat, so daß bei den Isoidid- und Isomannid-Derivaten jeweils die Monoacyl-Verbindung vom Diacylat abgetrennt werden muß, während beim Isosorbid, bei dem zwei stereoisomere Monoacyl-Derivate neben dem Diacylat entstehen, das gewünschte Acylat aus dem Gemisch der drei Acyl-Derivate isoliert werden muß. Die auftrennung des Acylatgemisches erfolgt entweder durch fraktionierte Kristallisation, fraktionierte Extraktion oder mit Hilfe anderer an sich bekannter Methoden.

Die mühsame und aufwendige Auftrennung des Acylat-Gemisches entfällt jedoch bei Anwendung eines weiteren erfindungesgemäßen Syntheseweges dadurch, daß 1.4;3.6-Dianhydro-D-glucit mit einem Überschuß an Sulfonsäurechlorid, vorzugsweise Methansulfonylchlorid oder Toluolsulfonylchlorid, in Pyridin oder Chloroform/Triäthylamin quantitativ zum entsprechenden 1.4;3.6-Dianhydro-D-glucit-2.5-diacylat umgesetzt wird.

Das Diacylat wird dann unter entsprechenden Bedingungen, wie bei dem ersten Syntheseweg beschrieben, der Aminolyse unterworfen, wobei infolge bevorzugter Substitution der 5-endo-Acylgruppe unter Konfigurationsumkehr und infolge teilweiser Hydrolyse der 2-exo-Acylgruppe unter Biebehaltung der Konfiguration neben einer geringen, in der wäßrigen Phase verbleibenden Menge an 2.5 - Diamino - 2.5 - didesoxy - 1.4;3.6 - dianhydro - D - glucit ein Gemisch aus 5 - Amino - 5 - desoxy - 1.4;3.6 - dianhydro - L - idit und 5 - Amino - 5 - desoxy - 1.4;3.6 - dianhydro - L - idit - 2 - methansulfonat im Verhältnis von etwa 1:4 entsteht.

Zur Vervollständigung der Hydrolyse der 2-exo-Acylgruppe wird denn dieses 1:4-Gemisch einer alkalischen oder sauren Hydrolyse unterworfen, und der entstehende 5 - Amino - 5 - desoxy - 1.4;3.6 - dianhydro - L - idit wird anschließend gegebenenfalls mit dem gewünschten reaktiven Purinyl-alkyl-Derivat kondensiert.

Wie sich aus der Tatsache, daß die Aminolyse des Isosorbid-2.5-diacylats überwiegend zum 5-Amino-isoidid-2-acylat führt, ergibt, ist die nucleophile Substitution der 2-exo-Acylatgruppe im Isosorbid-diacylat sterisch gehindert. Der Grad der sterischen Hinderung ist temperaturabhängig. Um aus dem entsprechenden Diacylat möglichst quantitativ das 5-Amino-isoidid-2-acylat zu erhalten, arbeitet man deshalb erfindungsgemäß vorzugsweise bei einer Temperatur von 80 bis 110°C, da bei Temperaturen über 110°C auch die 2-exo-Acylgruppe, wenn auch in geringem Ausmaß, durch Ammoniak oder durch das eingesetzte Amin angegriffen wird. Als Lösungsvermittler kann der wäßrigen Ammoniaklösung oder dem Amin ein Alkohol, vorzugsweise Äthanol, zugesetzt werden. Im Falle der Aminolyse durch Purin-Alkalisalze

verwendet man dipolare aprotische Lösungsmittel, vorzugsweise Dimethylsulfoxid, Dimethylformamid oder Diäther von Mono-, Di- oder Polyäthylenglykol.

Bei einem weiteren erfindungsgemäßen Weg zur Herstellung der erfindungsgemäßen Verbindungen macht man sich die überraschend aufgefundene Tatsache zunutze, daß 1.4;3.6 - Dianhydro - D - glucit - 2.5 - diacylate (Dimesylat oder Ditosylat) von Natriumbenzoat oder anderen Alkalisalzen der Benzoesäure in einem geeigneten Lösungsmittel, vorzugsweise einem dipolaren aprotischen Lösungsmittel, beispielsweise in wasserfreiem Dimethylformamid, Dimethylsulfoxid oder Diäthern des Äthylenglykols bei Temperaturen von 100 bis 180°, vorzugsweise 120 bis 150°C selektiv am $C^5$-Atom angegriffen werden, so daß unter Konfigurationsumkehr in hoher Ausbeute 1.4;3.6 - Dianhydro - L - idit - 2 - methansulfonat - 5 - benzoat bzw. 1.4;3.6 - Dianhydro - L - idit - 2 - toluolsulfonat - 5 - benzoat entsteht. Dieses Produkt wird nun wieder der Ammonolyse mit 25%iger Ammoniaklösung oder der Aminolyse unter erhöhtem Druck und erhöhter Temperatur unterworfen, wobei der Benzoesäureester nicht substituiert, sondern hydrolytisch abgespalten wird, und zwar unter Beibehaltung der Konfiguration am $C^5$-Atom, während der Acylatrest am $C^2$-Atom unter Konfigurationsumkehr durch die Amino- bzw. Alkylaminogruppe zum entsprechenden 5 - Amino - 5 - desoxy - 1.4;3.6 - dianhydro - D - glucit bzw. 5 - Alkylamino - 5 - desoxy - 1.4;3.6 - dianhydro - D - glucit substituiert wird.

Da das zuletzt genannte elegante Verfahren mit einer zweimaligen selektiven Konfigurationsumkehr verbunden ist, ist die Konfiguration des Endproduktes bezüglich seiner Substituenten identisch mit der Konfiguration der Ausgangsverbindung; aus dem Isosorbid-disulfonat entsteht wieder ein Isosorbid-Derivat, nämlich 5-Amino- bzw. 5 - Alkylamino - 5 - desoxy - 1.4;3.6 - dianhydro - D - glucit.

Die so gewonnenen 5-Amino- bzw. 5-Alkylamino-5-desoxy-1.4;3.6-dianhydro-D-glucit-Derivate können anschließend nach den zuvor beschriebenen Reaktionswegen mit reaktiven Purinylalkylderivaten zu den erfindungsgemäßen 5 - (ω - Purinyl - alkylamino) - 5 - desoxy - 1.4;3.6 - dianhydro - D - glucit - Derivaten kondensiert werden.

Anstelle der freien Basen können ggf. auch die Säureadditionssalze der erfindungsgemäßen Verbindungen eingesetzt werden, und zwar die Additionssalze anorganischer Säuren und Mineralsäuren, beispielsweise der Halogenwasserstoffsäuren, Schwefelsäure oder Phosphorsäuren, sowie organischer Säuren, beispielsweise der Carbon- und Sulfonsäuren. Die freien Basen können aus den Säureadditionssalzen wieder durch Behandlung mit starken Basen, beispielsweise Natrium- oder Kaliumhydroxid, freigesetzt werden.

Die in den folgenden Beispielen enthaltenen Abkürzungen haben folgende Bedeutungen:

| | |
|---|---|
| Schmp. | =Schmelzpunkt (unkorrigiert) |
| (Z) | =Zersetzung |
| d | =Dichte |
| $[\alpha]_D^{25}$ | =optische Drehung bei 25°C, Natrium-D-Linie. |

Hinter den optischen Drehwerten sind die Konzentrationen der gemessenen Lösungen angegeben, wobei c 2 beispielsweise eine Konzentration von 2 g/100 ml Lösung bedeutet; das Lösungsmittel ist jeweils gesondert angegeben. Alle Temperaturen sind in Grad Celsius angegeben.

Beispiel Nr. 1:

5-Amino-5-desoxy-1.4;3.6-dianhydro-L-idit:

a) 1.4;3.6-Dianhydro-D-glucit-2-methansulfonat, -5-methansulfonat und 2.5-dimethansulfonat:

Zur Lösung von 4.82 kg (33 Mol) 1.4;3.6-Dianhydro-D-glucit in 24 Liter Pyridin tropft man unter Feuchtigkeitsausschluß, Rühren und Kühlen auf −15° bis −20° innerhalb mehrerer Stunden 3.1 Liter (40 Mol) Methansulfonsäurechlorid. Anschließend rührt man 15 Stunden ohne Kühlung weiter. Man destilliert im Vak. das Pyridin ab, gibt zum öligen Rückstand 15 Liter Wasser, kocht auf und läßt abkühlen. Absaugen, Waschen mit 4 Litern Wasser und Trocknen des kristallinen Niederschlags ergibt 2.22 kg (7.34 Mol) 1.4;3.6 - Dianhydro - D - glucit - 2.5 - dimethansulfonat. Das Filtrat wird unter Rühren und Wasserkühlung mit ca. 1.5 kg Natriumhydroxid neutralisiert und bei ca. 70° im Vakuum bis zur Trockne eingedampft. Der Trockenrückstand wird mit insgesamt 30 Litern Chloroform kontinuierlich heiß extrahiert und der Extrakt heiß filtriert. Man läßt den Extrakt 15 Stdn. bei 20° stehen, saugt den kristallinen Niederschlag ab, wäscht ihn zweimal mit je 2 Litern Chloroform, trocknet und erhält 2.3 kg (10.26 Mol) 1.4;3.6 - Dianhydro - D - glucit - 5 - methansulfonat. Die vereinigten Filtrate werden im Vakuum eingedampft und der Rückstand heiß in 22 Litern Aethanol gelöst. Man läßt 15 Stunden bei 20° stehen, saugt den kristallinen Niederschlag ab, wäscht ihn zweimal mit je 3 Litern Aethanol, trocknet und erhält 0.65 kg (2.90 Mol) 1.4;3.6 - Dianhydro - D - glucit - 2 - methansulfonat. Eindampfen der Filtrate ergibt 2.21 kg (9.85 Mol) eines Gemisches der beiden isomeren Mono-methansulfonate, das je nach Bedarf durch Wiederholung der abwechselnden Kristallisationen aus Chloroform und Aethanol weiter aufgetrennt werden kann, bzw. durch Veresterung mit Methansulfonsäurechlorid in Pyridin vollständig in 1.4;3.6 - Dianhydro - D - glucit - 2.5 - dimethansulfonat übergeführt wird.

Analytische Mengen der Methansulfonate ergeben nach Umkristallisation korrekte Elementaranalysen und die in Tabelle 1 aufgeführten Schmelzpunkte und optischen Drehungen:

TABELLE 1

| 1.4;3.6-Dianhydro-D-glucit- | Umkristallisation aus | Schmp. [°C] | $[\alpha]_D^{25}$ |
|---|---|---|---|
| -2-methansulfonat | Chloroform | 135—138.5 | 62.5 (c 2; Aceton) |
| -5-methansulfonat | Chloroform | 123—124 | 75.9 (c 2; Methanol) |
| -2.5-dimethansulfonat | Aethanol/Aceton | 127—128 | 74 (c 2; Aceton) |

Anmerkung:

Setzt man 1.4;3.6-Dianhydro-D-glucit mit der 2 bis 2.5-fach molaren Menge Methansulfonsäurechlorid unter den gleichen Reaktionsbedingungen um, erhält man das 1.4;3.6-Dianhydro-D-glucit-2.5-dimethansulfonat in nahezu quantitativer Ausbeute.

b) 5-Amino-5-desoxy-1.4;3.6-dianhydro-L-idit:

Das Produkt kann auf folgenden 2 Wegen erhalten werden:

Verfahren 1:

Herstellung durch Ammonolyse von 1.4;3.6-Dianhydro-D-glucit-5-methansulfonat. Das Gemisch aus 448 g (2 Mol) 1.4;3.6-Dianhydro - D - glucit - 5 - methansulfonat und 1.5 Liter 25 %igem wäßrigen Ammoniak (20 Mol) wird im geschlossenen Stahlautoklaven 24 Stunden bei 130° gerührt. Danach ist die Umsetzung quantitativ. Man dampft unter vermindertem Druck ein und trocknet azeotrop durch aufeinanderfolgende Zugabe und erneutes Eindampfen von je 1 Liter Aethanol und Chloroform nach. Der ölige Rückstand wird unter Erwärmen in 500 ml Aethanol gelöst und mit Isopropanol auf 2 Liter verdünnt. Beim Erkalten kristallisieren 311 g (1.3 Mol) 5 - Amino - 5 - desoxy - 1.4;3.6 - dianhydro - L - idit als methansulfonsaures Salz aus. Weitere 100 g (0.4 Mol) kristallines Reinprodukt fallen nach Behandeln der Mutterlauge mit 30 g Aktivkohle und Konzentrieren des Filtrats aus. Zur Analyse kristallisiert man aus Aethanol/Chloroform um.

Schmp. 151—4°; $[\alpha]_D^{25}$ 27.6 (c 1; Wasser)

Elementaranalyse: $C_6H_{11}NO_3 \times CH_3SO_3H$ (241.27)

Ber.: C (34.83), H (6.27), N (5.81)

Gef.: C (34.71), H (6.45), N (5.36)

Ein kleiner Teil des Produkts wird in die freie Base übergeführt und aus Chloroform/Aether umkristallisiert. Schmp. 103—104°; $[\alpha]_D^{25}$ 31.6 (c 2; Wasser)

Verfahren 2:

Ammonolyse von 1.4;3.6-Dianhydro-D-glucit-2.5-dimethansulfonat gefolgt von alkalischer Hydrolyse des erhaltenen 5 - Amino - 5 - desoxy - 1.4;3.6 - dianhydro - L - idit - 2 - methansulfonats.

Das Gemisch aus 302 g (1 Mol) 1.4;3.6-Dianhydro-D-glucit-2.5-dimethansulfonat, 750 ml 25 %igem wäßrigen Ammoniak (10 Mol) und 750 ml Aethanol wird im verschlessenen Stahlautoklaven 4 Tage bei 100° gerührt Nach dem Abkühlen versetzt man mit 1 Liter Wasser und saugt vom auskristallisierten nicht umgesetzten Dimethansulfonat (100 g=0.35 Mol) ab. Das Filtrat wird zur Entfernung von Ammoniak mit 104 g (1.3 Mol) Natriumhydrogencarbonat versetzt und unter vermindertem Druck eingedampft. Man löst in 5 Liter Wasser und extrahiert mit 500 ml Chloroform Eliminierungsprodukte heraus. Die wäßrige Phase wird 48 Stdn. lang im Rotationsperforator (Normag) kontinuierlich mit Chloroform extrahiert. In der Wasserphase verbleibt als Nebenprodukt entstandener 2.5 - Diamino - 2.5 - didesoxy - 1.4;3.6 - dianhydro - D - glucit. Der Chloroformextrakt ergibt nach dem Trocknen über wasserfreiem Natriumsulfat, Filtrieren und Eindampfen 105 g (ca. 0.55 Mol) eines 1:4-Gemisches aus 5 - Amino - 5 - desoxy - 1.4;3.6 - dianhydro - L - idit und 5 - Amino - 5 - desoxy - 1.4;3.6 - dianhydro - L - idit - 2 - methansulfonat.

Zur Charakterisierung des letzteren Produkts wird ein kleiner Teil des Gemisches in Chloroform gelöst, 2 mal mit Wasser gewaschen, die Chloroformphase eingedampft, in das methansulfonsaure Salz übergeführt und 2 mal aus Aethanol umkristallisiert.

Schmp. 213—5°; $[\alpha]_D^{25}$ 39.0 (c 0.50; Wasser)

Elementaranalyse: $C_7H_{13}NO_5S \times CH_3SO_3H$ (319.37)

Ber.: C (30.09), H (5.37), N (4.39), S (20.08)

Gef.: C (30.13), H (5.49), N (4.25), S (20.6)

Das oben erhaltene Gemisch wird zu einer Lösung von 60 g (1.5 Mol) Natriumhydroxid in 1.5 Litern Wasser gegeben und 24 Stunden unter Rückfluß gekocht. Nach dem Abkühlen stellt man durch Zugabe von konz. Salzsäure auf pH=10 ein, filtriert und dampft unter vermindertem Druck ein, trocknet aceotrop mit n-Butanol nach, erwärmt den Rückstand mit 500 ml n-Butanol und filtriert von anorganischen Salzen ab. Die butanolische Lösung wird eingedampft, der Rückstand in 200 ml Isopropanol gelöst und mit 34 g (0.35 Mol) Methansulfonsäure versetzt. Es kristallisieren 80 g (0.33 Mol) 5 - Amino - 5 - desoxy - 1.4;3.6 -

dianhydro - L - idit in Form des methansulfonsauren Salzes aus. Schmp. 150—2°. Ausbeute bezogen auf umgesetztes 1.4;3.6 - Dianhydro - D - glucit - 2.5 - dimethansulfonat: 50%

Beispiel Nr. 2:
5-Amino-5-desoxy-1.4;3.6-dianhydro-L-idit:

Zur Lösung von 2923 g (20 Mol) 1.4;3.6-Dianhydro-D-glucit in 16 Litern wasserfr. Pyridin tropft man unter Rühren und Kühlen auf −15° 2 Liter (25 Mol) Methansulfonsäurechlorid, rührt 15 Stdn. bei −15° nach und destilliert das Pyridin unter vermindertem Druck ab. Der ölige Rückstand wird mit 10 Litern Wasser versetzt, kurz zum Sieden erhitzt und nach dem Abkühlen vom auskristallisierten 1.4;3.6 - Dianhydro - D - glucit - 2.5 - dimethansulfonat abfiltriert und der Filterkuchen 2 mal mit je 2.5 Litern Wasser gewaschen. Die vereinigten Filtrate werden nach Zugabe von 1 kg (25 Mol) Natriumhydroxid unter reduziertem Druck eingedampft. Der Rückstand, bestehend aus dem Gemisch von 1.4;3.6-Dianhydro-D-glucit-2- und -5-methansulfonat und Natriummethansulfonat wird ohne weitere Reinigung der wäßrigen Ammonolyse unterworfen. Hierzu versetzt man mit 4 Litern Wasser und 12 Litern 25 %igem wäßrigem Ammoniak und erhitzt die so erhaltene Lösung 2 Tage unter Rühren im geschlossenen Stahlautoklaven auf 120° (Druck: ca. 7—8 bar). Nach dem Abkühlen und Entspannen setzt man 400 g Aktivkohle zu, filtriert, engt das Filtrat auf ca. 4 Liter ein und extrehiert zweimal mit je 1 Liter Chloroform den als Nebenprodukt aus dem 1.4;3.6 - Dianhydro - D - glucit - 2 - methansulfonat gebildeten 1.4;2.5;3.6 - Trianhydro - D - mannit (insgesamt ca. 90 g=0.7 Mol) heraus. Die wäßrige Phase wird durch Zugabe von Natriumhydroxid auf pH=9 gebracht und zur Trockne eingedampft. Der halbfeste Rückstand wird zur Abtrennung anorganischen Materials mit 10 Litern Aethanol aufgekocht und nach dem Abkühlen filtriert. Das eingedampfte Filtrat wird in 10 Litern n-Butanol unter Erwärmen gelöst, über wasserfr. Natriumsulfat getrocknet, filtriert, eingedampft und im Vakuum fraktionierend destilliert. Nach dem Vorlauf (0.4 Torr. 122—135°) von 55 g (0.38 Mol) durch Trianhydro-D-mannit schwach verunreinigtem 5 - Amino - 5 - desoxy - 1.4;3.6 - dianhydro - L - idit destillieren zwischen 140° und 144° bei 0.15 bis 0.2 Torr insgesamt 681 g (4.69 Mol) reiner 5 - Amino - 5 - desoxy - 1.4;3.6 - dianhydro - L - idit als langsam erstarrendes schwach gelbliches Oel über. Schmp. (nach Umkristallisation aus Chloroform/Aethylacetat/Aether) 103—4°; $[\alpha]_D^{25}$ 31.6 (c 2.0; Wasser).

Beispiel Nr. 3:
5-Amino-5-desoxy-1.4;3.6-dianhydro-D-glucit:

Das Produkt kann auf folgenden zwei Wegen erhalten werden:

Verfahren 1:

Herstellung und Ammonolyse von 1.4;3.6-Dianhydro-L-idit-2-methansulfonat.

Die Lösung von 73 g (480 mmol) 96 %ig. 1.4;3.6-Dianhydro-L-idit in 500 ml wasserfreiem Pyridin wird unter Feuchtigkeitsauschluß, Rühren und Kühlen auf −20° tropfenweise mit 52 ml (660 mmol) 98 %igem Methansulfonylchlorid versetzt und 15 Stdn. bei −20° nachgerührt. Unter vermindertem Druck destilliert man das Pyridin soweit wie möglich ab und erwärmt den Rückstand nach Zugabe von 500 ml heißem Wasser bis zur Lösung. Beim Abkühlen kristallisieren 47.6 g (157 mmol) 1.4;3.6 - Dianhydro - L - idit - 2.5 - dimethansulfonat aus, die abgesaugt und zweimal mit je 100 ml Wasser nachgewaschen werden. Die vereinigten Filtrate werden durch Zugabe von Natriumhydrogencarbonat neutralisiert (pH=7), unter vermindertem Druck eingedampft und getrocknet. Der gepulverte Trockenrückstand wird zweimal mit je 400 ml Chloroform ausgekocht und noch heiß filtriert. Nach dem Abkühlen des Filtrats kristallisiert 1.4;3.6 - Dianhydro - L - idit - 2 - methansulfonat aus. Die Mutterlauge liefert nach Konzentration weiteres Monomethansulfonat. Insgesamt erhält man 51.5 g (213 mmol) 1.4;3.6 - Dianhydro - L - idit - 2 - methansulfonat. Zur Analyse wird eine kleine Menge aus Methanol umkristallisiert.

Schmp. 124—5°; $[\alpha]_D^{25}$ 33.7 (c 1.0; Aceton).

Elementaranalyse: $C_7H_{12}O_6S$ (224.24)

Ber.: C (37.50), H (5.40), S (14.30)

Gef.: C (37.58), H (5.53), S (14.0)

33.6 g (150 mmol) des so erhaltenen 1.4;3.6-Dianhydro-L-idit-2-methansulfonats werden zusammen mit einer Lösung von 17 g (1 Mol) Ammoniak in 250 ml n-Butanol im geschlossenen Stahlautoklaven 3 Tage auf 170° erhitzt. Nach dem Abkühlen saugt man auskristallisiertes Ammoniummethansulfonat abt und wäscht mit 100 ml n-Butanol nach. Das Filtrat wird zweimal mit je 200 ml Wasser extrahiert. Die vereinigten wäßrigen Extrakte werden mit 200 ml Chloroform gewaschen, zur Trockne eingedampft und aceotrop mit Butanol nachgetrocknet. Der Trockenrückstand wird unter Zusatz von 10 g wasserfreiem Natriumsulfat mit 50 ml n-Butanol aufgekocht, heiß filtriert und das Filtrat eingedampft. Das so erhaltene ölige Rohprodukt wird in 50 ml Chloroform aufgenommen, filtriert und eingedampft. Man erhält 14 g (96 mmol) langsam erstarrenden 5 - Amino - 5 - desoxy - 1.4;3.6 - dianhydro - D - glucit. Zur Charakterisierung überführt man einen kleinen Anteil des Produkts in das Hydrochlorid und kristallisiert aus Isopropanol um.

Zersetzungspunkt: 240°; $[\alpha]_D^{25}$ 39.1 (c 1.0; Wasser)

Elementaranalyse: $C_6H_{11}NO_3 \times HCl$ (181.63)

Ber.: C (39.68), H (6.66), N (7.71), Cl (19.52)

Gef.: C (39.85), H (6.87), N (7.66), Cl (19.3)

Verfahren 2:

Herstellung und selektive Ammonolyse von 1.4;3.6-Dianhydro-L-idit-2-methansulfonat-5-benzoat.

Die Mischung aus 604 g (2 Mol) 1.4;3.6-Dianhydro-D-glucit-2.5-dimethansulfonat, 317 g (2.2 Mol) Natriumbenzoat und 8 Litern wasserfreiem Dimethylformamid wird 2 Tage bei 145° im Stahlautoklaven unter Stickstoffschutzatmosphäre gerührt. Unter vermindertem Druck destilliert man das Dimethylformamid ab, nimmt den Rückstand in 5 Litern Chloroform auf, extrahiert nacheinander mit je 2 Litern 1-molarer Natronlauge und Wasser, trocknet die Chloroformphase über wasserfreiem Natriumsulfat, filtriert und konzentriert auf 1500 ml Volumen. Das beim Stehenlassen kristallisierende Rohprodukt wird abgesaugt, unter Erwärmen in 500 ml Aceton gelöst und die heiße Lösung in 1000 ml Aethanol gegossen. Beim Abkühlen kristallisieren 273 g (0.83 Mol) 1.4;3.6 - Dianhydro - L - idit - 2 - methansulfonat - 5 - benzoat aus. Die Mutterlauge ergibt nach Eindampfen und Umristallisation weitere 120 g (0.37 Mol) schwach durch Ausgangssubstanz verunreinigtes Produkt. Die analytische Probe hat nach Umkristallisation aus Aethanol den Schmp.

117° und $[\alpha]_D^{25}$ 76.6 (c 2; Chloroform).

Elementaranalyse: $C_{14}H_{16}O_7S$ (328.35)

Ber.: C (51.21), H (4.91), S (9.76)

Gef.: C (51.60), H (5.05), S (9.6)

328 g (1 Mol) des so erhaltenen 1.4;3.6-Dianhydro-L-idit-2-methansulfonat-5-benzoats werden zusammen mit 1 Liter Aethanol und 1.5 Liter 25 %igem wäßrigen Ammoniak 1 Tag bei 130° im geschlossenen Stahlautoklaven gerührt. Man dampft unter vermindertem Druck ein, löst den Rückstand in 1 Liter Wasser, stellt durch Zugabe von konz. Salzsäure auf pH=1 ein und saugt den Niederschlag—bestehend aus Benzoesäure und Benzamid—ab. Das Filtrat wird nach zweimaligem Waschen mit je 500 ml Chloroform durch Zugabe von Natriumhydrogencarbonat auf pH=8 gebracht, erneut eingedampft und der Rückstand mit 2 Litern Aethanol extrahiert. Der Aethanol-Extrakt wird nach dem Eindampfen mit 2 Litern Chloroform extrahiert, der Chloroformextrakt mit 60 g Aktivkohle aufgekocht, filtriert und eingedampft. Die so erhaltenen 105 g Rohprodukt liefern nach fraktionierter Destillation bei 0.2 Torr und 136—142° Uebergangstemperatur 86.3 g (0.59 Mol) 5 - Amino - 5 - desoxy - 1.4;3.6 - dianhydro - D - glucit in Form eines langsam erstarrenden farblosen Oels, das nach Ueberführung in das Hydrochlorid identisch mit dem nach Verfahren 1 gewonnenen Produkt ist.

Beispiel Nr. 4:

2-Amino-2-desoxy-1.4;3.6-dianhydro-D-glucit:

a) 1.4;3.6-Dianhydro-D-mannit-2-methansulfonat:

Der Lösung von 877 g (6 Mol) 1.4;3.6-Dianhydro-D-mannit in 6 Litern Pyridin tropft man unter Rühren und Feuchtigkeitsausschluß sowie Kühlen auf −15° innerhalb 6 Stdn. 525 ml (6.6 Mol) Methansulfonylchlorid zu, rührt weitere 3 Tage bei −15° und destilliert dann unter vermindertem Druck das Pyridin ab. Beim Versetzen des öligen Rückstande mit 2.7 Litern Wasser kristallisiert reines 1.4;3.6 - Dianhydro - D - mannit - 2.5 - dimethansulfonat aus, das abgetrennt und 2 mal mit je 700 ml Wasser gewaschen wird. Die vereinigten Filtrate werden mit einer Lösung von 264 g (6.6 Mol) Natriumhydroxid in 2.5 Litern Wasser versetzt, durch Zugabe von Natriumhydrogencarbonat auf pH=7 eingestellt, unter reduziertem Druck eingedampft und aceotrop mit Chloroform getrocknet. Der Rückstand wird zweimal mit je 2.5 Litern Chloroform heiß extrahiert und filtriert. Die vereinigten Chloroformextrakte werden 5 mal mit je 1 Liter Wasser extrahiert. Beim Konzentrieren der Wasserphasen kristallisiert das 1.4;3.6 - Dianhydro - D - mannit - 2 - methansulfonat aus. Die nach dem Absaugen verbleibende Mutterlauge ergibt nach dem Eindampfen und Umkristallisieren aus Aethanol weiteres Produkt. Restliches Produkt wird durch Eindampfen der aethanolischen Mutterlauge, Lösen des Rückstandes in Wasser und kontinuierliche Extraktion der wäßrigen Lösung mit Chloroform im Rotationsperforator gewonnen. In der Wasserphase verbleibt nicht umgesetzter 1.4;3.6-Dianhydro-D-mannit. Insgesamt er hält man 396 g (1.77 Mol) 1.4;3.6 - Dianhydro - D - mannit - 2 - methansulfonat (neben 465 g=1.54 Mol des Dimethansulfonats). Die analytische Probe hat nach Umkristallisation aus Chloroform den Schmp. 111—112° und $[\alpha]_D^{25}$ 118 (c 1.0; Aceton).

Elementaranalyse: $C_7H_{12}O_6S$ (224.24)

Ber.: C (37.50), H (5.40), S (14.30)

Gef.: C (37.41), H (5.59), S (13.7)

b) 2-Amino-2-desoxy-1.4;3.6-dianhydro-D-glucit:

Die Mischung aus 224 g (1 Mol) des zuvor erhaltenen 1.4;3.6-Dianhydro-D-mannit-2-methansulfonats und 1 Liter 25 %igem wäßrigen Ammoniak wird 24 Stdn. bei 120° im geschlossenen Stahlautoklaven gerührt. Nach dem Abkühlen gibt man 84 g (1 Mol) Natriumhydrogencarbonat zu, dampft unter reduziertem Druck ein und kocht den Rückstand mit 2 Litern n-Butanol aus. Der eingedampfte Butanol-Extrakt wird in 1 Liter Chloroform aufgenommen, restliches Natriummethansulfonat abriltriert und das Filtrat eingedampft. Man erhält 130 g (0.9 Mol) 2 - Amino - 2 - desoxy - 1.4;3.6 - dianhydro - D - glucit als schwach gelbliches Oel. Zur Charakterisierung überführt man einen kleinen Teil in das Hydrochlorid und kristallisiert aus Isopropanol/Methanol/Chloroform um.

Schmp. 230° (Z); $[\alpha]_D^{25}$ 52.1 (c 1.0; Wasser)
Elementaranalyse: $C_6H_{11}NO_3 \times HCl$ (181.62)
Ber.: C (39.68), H (6.66), N (7.71), Cl (19.52)
Gef.: C (39.59), H (6.89), N (7.52), Cl (19.3)

Beispiel Nr. 5:
2-Amino-2-desoxy-1.4;3.6-dianhydro-D-mannit:

Das Gemisch aus 448 g (2 Mol) 1.4;3.6-Dianhydro-D-glucit-2-methansulfonat (Herstellung siehe Beispiel 1 a) und 1500 ml 25 %igem wäßrigen Ammoniak wird 1 Tag bei 130° im geschlossenen Stahlautoklaven gerührt. Nach dem Abkühlen gibt man 30 g Aktivkohle zu, filtriert und extrahiert mit 1 Liter Chloroform den als Nebenprodukt gebildeten 1.4;2.5;3.6-Trianhydro-D-mannit [nach Eindampfen der Chloroformphase und Umkristallisation aus Aether/Petroläther insgesamt 104 g (0.81 Mol)]. Die Wasserphase wird nach dem Eindampfen unter reduziertem Druck und aceotropem Trocknen mit Aethanol und Chloroform in der Siedehitze mit 2 Litern Isopropanol extrahiert. Beim Einengen des Isopropanolextrakts auf 0.5 Liter auskristallisierendes Ammoniummethansulfonat wird abfiltriert, das Filtrat mit verdünnter Natronlauge neutralisiert, eingedampft und mit 1 Liter n-Butanol heiß extrahiert. Der Butanolextrakt wird eingedampft und der Rückstand mit 1 Liter Chloroform extrahiert. Eindampfen des filtrierten Chloroformextrakts ergibt 60 g (0.41 Mol) ölige Rohbase, die in 100 ml Essigsäure gelöst und tropfenweise mit einer Lösung von 15 ml 96 %iger Salpetersäure (d=1.5) in 75 ml Essigsäure versetzt wird. Das auskristallisierende Hydrogennitrat wird abgesaugt und aus Isopropanol/Aethanol umkristallisiert. Man erhält 32 g (154 mmol) 2 - Amino - 2 - desoxy - 1.4;3.6 - dianhydro - D - mannit - Hydrogennitrat.

Schmp. 192—3° (Z); $[\alpha]_D^{25}$ 63.4 (c 0.51; Wasser)
Elementaranalyse: $C_6H_{11}NO_3 \times HNO_3$ (208.18)
Ber.: C (34.62), H (5.81), N (13.45)
Gef.: C (34.52), H (5.97), N (13.53)

Ein kleiner Teil wird in das Hydrochlorid übergeführt und aus Aethanol umkristallisiert.

Schmp. 263—8° (Z); $[\alpha]_D^{25}$ 77.8 (c 1; Wasser)
Elementaranalyse: $C_6H_{11}NO_3 \times HCl$ (181.63)
Ber.: C (39.68), H (6.66), N (7.71), Cl (19.52)
Gef.: C (39.82), H (6.68), N (7.59), Cl (19.4)

Beispiel Nr. 6:
5-Methylamino-5-desoxy-1.4;3.6-dianhydro-L-idit:

Die Mischung aus 22.4 g (0.1 Mol) 1.4;3.6-Dianhydro-D-glucit-5-methansulfonat (Herstellung siehe Beispiel 1 a), 31 g (1 Mol) Methylamin und 150 ml n-Butanol wird im geschlossenen Stahlautoklaven 15 Stdn. bei 150° unter Stickstoffatmosphäre gerührt. Nach dem Abkühlen setzt man die Lösung von 4 g (0.1 Mol) Natriumhydroxid in 200 ml n-Butanol zu, rührt durch, fällt mit 600 ml Chloroform das gebildete Natriummethansulfonat aus, filtriert und dampft das Filtrat unter vermindertem Druck ein. Die so erhaltene ölige Rohbase wird in 100 ml Isopropanol gelöst und mit 6.5 ml 65 %iger Salpetersäure in das Hydrogennitrat übergeführt. Nach dem Eindampfen unter reduziertem Druck kristallisiert man aus Isopropanol um und erhält 15.3 g (68.9 mmol) 5 - Methylamino - 5 - desoxy - 1.4;3.6 - dianhydro - L - idit - Hydrogennitrat.

Schmp. 103—9°; $[\alpha]_D^{25}$ 41.8 (c 1.0; Wasser)
Elementaranalyse: $C_7H_{13}NO_3 \times HNO_3$ (222.20)
Ber.: C (37.84), H (6.35), N (12.61)
Gef.: C (38.00), H (6.60), N (12.23)

Beispiel Nr. 7:
5-Aethylamino-5-desoxy-1.4;3.6-dianhydro-L-idit:

Das Gemisch aus 11.2 g (50 mmol) 1.4;3.6-Dianhydro-D-glucit-5-methansulfonat, 40.5 g (500 mmol) Aethylamin-Hydrochlorid, 20 g (500 mmol) Natriumhydroxid und 200 ml Wasser wird im geschlossenen Stahlautoklaven 15 Stdn. bei 150° unter Stickstoffatmosphäre gerührt. Nach dem Abkühlen und Entspannen gibt man 2 g (50 mmol) Natriumhydroxid zu, engt unter reduziertem Druck zur Trockne ein, extrahiert den Rückstand heiß mit Chloroform und dampft den Extrakt zur Trockne ein. Man erhält 9 g (ca. 50 mmol) öligen 5 - Aethylamino - 5 - desoxy - 1.4;3.6 - dianhydro - L - idit, der für die nachfolgende Veresterung mit Salpetersäure eingesetzt wird. Zur Charakterisierung wird ein kleiner Anteil in das Hydrochlorid übergeführt und 2 mal aus Isopropanol umkristallisiert.

Schmp. 181—3° (Z); $[\alpha]_D^{25}$ 51.3 (c 1; Wasser)
Elementaranalyse: $C_8H_{15}NO_3 \times HCl$ (209.68)
Ber.: (45.83), H (7.69), N (6.68), Cl (16.91)
Gef.: C (46.26), H (8.06), N (6.69), Cl (16.9)

Beispiel Nr. 8:
5-Adamant-1-ylamino-5-desoxy-1.4;3.6-dianhydro-L-idit:

Das Gemisch aus 11.2 g (50 mmol) 1.4;3.6-Dianhydro-D-glucit-5-methansulfonat, 39 g (250 mmol) 1-Aminoadamantan und 100 ml n-Butanol wird 3 Tage im geschlossenen Stahlautoklaven auf 150° erhitzt.

Anschließend dampft man unter reduziertem Druck zur Trockne ein, löst den Rückstand in 250 ml Chloroform, gewinnt durch Extraktion mit 1-molarer Salzsäure zunächst überschüssiges Adamantylamin zurück und bei weiterer Extraktion das Reaktionprodukt. Die salzsaure Lösung des Reaktionsproduktes wird mit verdünnter Natronlauge auf pH=8 gestellt und mit Chloroform reextrahiert. Eindampfen der über wasserfr. Natriumsulfat getrockneten Chloroformphasen liefert 3.85 g (13.8 mmol) 5 - Adamant - 1 - ylamino - 5 - desoxy - 1.4;3.6 - dianhydro - L - idit. Zur Analyse kristallisiert man aus Cyclohexan um.

Schmp. 126—7°; $[\alpha]_D^{25}$ 26.0 (c 1; Aethanol)
Elementaranalyse: $C_{16}H_{25}NO_3$ (279.39)
Ber.: C (68.79), H (9.02), N (5.01)
Gef.: C (68.80), H (9.25), N (5.07)

Beispiel Nr. 9:
5-Dimethylamino-5-desoxy-1.4;3.6-dianhydro-L-idit:

22.5 g (0.5 Mol) Dimethylamin werden unter Kühlen in 100 ml n-Butanol gelöst, mit 11.2 g (50 mmol) 1.4;3.6 - Dianhydro - D - glucit - 5 - methansulfonat versetzt und 15 Stdn. im geschlossenen Stahlautoklaven auf 150° erhitzt. Nach dem Abkühlen und Entspannen gibt man 100 ml 0.5-molare butanolische Natriumhydroxidlösung zu, filtriert und dampft unter reduziertem Druck ein. Der Rückstand wird mit Chloroform extrahiert und der Chloroformextrakt unter reduziertem Druck eingedampft. Man erhält 8.5 g (49 mmol) öligen 5 - Dimethylamino - 5 - desoxy - 1.4;3.6 - dianhydro - L - idit. Zur Charakterisierung überführt man einen kleinen Teil in das Hydrochlorid und kristallisiert aus Isopropanol um.

Schmp. 227—9°; $[\alpha]_D^{25}$ 46.8 (c 0.58; Wasser)
Elementaranalyse: $C_8H_{15}NO_3 \times HCl$ (209.68)
Ber.: C (45.82), H (7.69), N (6.68), Cl (16.91)
Gef.: C (46.02), H (7.96), N (6.79), Cl (16.6)

Beispiel Nr. 10:
5-Diäthylamino-5-desoxy-1.4;3.6-dianhydro-L-idit:

Herstellung analog Beispiel 9) durch Umsetzung von überschüssigem Diäthylamin mit 1.4;3.6-Dianhydro-D-glucit-5-methansulfonat in Butanol bei 150°. Isolierung als Hydrogennitrat.

Schmp. 129° (aus Isopropanol); $[\alpha]_D^{25}$ 39.8 (c 1; Wasser)
Elementaranalyse: $C_{10}H_{19}NO_3 \times HNO_3$ (264.28)
Ber.: C (45.45), H (7.63), N (10.60)
Gef.: C (44.97), H (7.86), N (10.65)

Beispiel Nr. 11:
5-Pyrrolidino-5-desoxy-1.4;3.6-dianhydro-L-idit:

Das Gemisch aus 11.2 g (50 mmol) 1.4;3.6-Dianhydro-D-glucit-5-methansulfonat, 36 g (0.5 Mol) Pyrrolidin und 100 ml n-Butanol wird im geschlossenem Stahlautoklaven unter Stickstoff-Schutzatmosphäre 24 Stdn. auf 150° erhitzt. Nach dem Abkühlen gibt man 10 ml 5-molare Natronlauge zu, dampft unter reduziertem Druck das überschüssige Pyrrolidin ab—gegen Ende unter Zusatz von Wasser—und extrahiert das Produkt aus dem Rückstand mit Chloroform heraus. Der eingedampfte Chloroformextrakt wird durch Lösen in Aether von unlöslichen Nebenprodukten befreit. Das Aetherfiltrat liefert nach Trocknen über wasserfreiem Natriumsulfat und Eindampfen 8,7 g (43.7 mmol) 5 - Pyrrolidino - 5 - desoxy - 1.4;3.6 - dianhydro - L - idit. Zur Charakterisierung wird ein kleiner Teil in das Hydrogennitrat übergeführt und aus Isopropanol umkristallisiert.

Schmp. 123—4°; $[\alpha]_D^{25}$ 45.9 (c 1; Aethanol)
Elementaranalyse: $C_{10}H_{17}NO_3 \times HNO_3$ (262.26)
Ber.: C (45.80), H (6.92), N (10.68)
Gef.: C (45.74), H (7.16), N (10.79)

Beispiel Nr. 12:
5-Piperidino-5-desoxy-1.4;3.6-dianhydro-L-idit:

Das Gemisch aus 11.2 g (50 mmol) 1.4;3.6-Dianhydro-D-glucit-5-methansulfonat, 42.5 g (0.5 Mol) Piperidin und 100 ml n-Butanol wird 24 Stdn. im geschlossenen Stahlautoklaven unter Stickstoffatmosphäre auf 150° erhitzt. Nach dem Eindampfen unter reduziertem Druck extrahiert man den Rückstand mit Aether, wobei Piperidin-Hydrogenmethansulfonat ungelöst bleibt. Der über wasserfr. Natriumsulfat getrocknete Aetherextrakt ergibt nach dem Eindampfen 10 g (47 mmol) Produkt, die in 50 ml Essigsäure gelöst und mit der äquimolaren Menge 30 %iger Salpetersäure in das Hydrogennitrat übergeführt werden. Dieses wird mit Aether ausgefällt und ergibt nach Umkristallisation aus Isopropanol 11.6 g (42 mmol) 5 - Piperidino - 5 - desoxy - 1.4;3.6 - dianhydro - L - idit - Hydrogennitrat.

Schmp. 182—4° (Z); $[\alpha]_D^{25}$ 46.0 (c 1; Aethanol)
Elementaranalyse: $C_{11}H_{19}NO_3 \times HNO_3$ (276.29)
Ber.: C (47.82), H (7.30), N (10.14)
Gef.: C (47.80), H (7.44), N (10.21)

Beispiel Nr. 13:

5-Morpholino-5-desoxy-1.4;3.6-dianhydro-L-idit:

Herstellung analog Beispiel 9) durch Umsetzung von 50 mmol 1.4;3.6-Dianhydro-D-glucit-5-methansulfonat mit 0.5 Mol Morpholin. Ausbeute 8.6 g (40 mmol) öliges Produkt. Charakterisierung als Hydrochlorid.

Schmp. 159—161° (aus Isopropanol/Aethanol). $[\alpha]_D^{25}$ 48.5 (c 1; Aethanol).

Elementaranalyse: $C_{10}H_{17}NO_4 \times HCl$ (251.71)

Ber.: C (47.72), H (7.21), N (5.57)

Gef.: C (47.42), H (7.20), N (5.61)

Beispiel Nr. 14:

5-(4-Methylpiperazino)-5-desoxy-1.4;3.6-dianhydro-L-idit:

Die Mischung aus 11.2 g (50 mmol) 1.4;3.6-Dianhydro-D-glucit-5-methansulfonat und 15 g (150 mmol) N-Methylpiperazin wird unter Stickstoff-Atomsphäre bei 150° 24 Stdn. im Stahlautoklaven gerührt. Nach dem Abkühlen gibt man eine Lösung von 2.0 g (50 mmol) Natriumhydroxid in 100 ml n-Butanol zu und dampft unter reduziertem Druck zur Trockne ein. Der ölige Rückstand wird mit 500 ml Chloroform behandelt und vom Ungelösten (Natriummethansulfonat) abfiltriert. Die Chloroformphase wird 2 mal mit je 200 ml Wasser extrahiert; die vereinigten Wasserphasen ergeben nach dem Eindampfen im Vak. und azeotrop. Trocknung mit Aethanol und Chloroform die Rohbase. Diese wird in 50 ml Eisessig gelöst und durch Zugabe der doppelt molaren Menge an verd. Salpetersäure in das Dihydrogennitrat übergeführt, das größtenteils direkt auskristallisiert. Restliches Produkt fällt man mit Aether aus. Umkristallisation aus Isopropanol/Aethanol ergibt 8.9 g (25 mmol) 5 - (4 - Methylpiperazino) - 5 - desoxy - 1.4;3.6 - dianhydro - L - idit - Dihydrogennitrat.

Schmp. 124—5° (Z): $[\alpha]_D^{25}$ 27.9 (c 0.5; Wasser)

Elementaranalyse: $C_{11}H_{20}N_2O_3 \times 2\ HNO_3$ (354.32)

Ber.: C (37.29), H (6.26), N (15.81)

Gef.: C (37.10), H (6.36), N (15.54)

Beispiel Nr. 15:

5-(9-Adenyl)-5-desoxy-1.4;3.6-dianhydro-L-idit:

Unter Stickstoffatmosphäre und Rühren tropft man bei 100° zur Suspension von 15.7 g (0.1 Mol) Adenin-Natriumsalz in 200 ml wasserfreiem Dimethylsulfoxid die Lösung von 22.4 g (0.1 Mol) 1.4;3.6 - Dianhydro - D - glucit - 5 - methansulfonat in 100 ml wasserfreiem Dimethylsulfoxid zu und rührt noch 8 Tage bei 100°. Man destilliert im Vakuum das Lösungsmittel ab und extrahiert den Rückstand in der Siedehitze nacheinander mit 100 ml Chloroform und n-Butanol. Der Chloroformextrakt wird auf ca. 300 ml konzentriert, durch Zugabe von 600 ml Petroläther das Rohprodukt ausgefällt und abgetrennt; das Filtrat wird verworfen. Der Butanolextrakt ergibt nach Eindampfen im Vak. weiteres Rohprodukt. Die vereinigten Rohprodukte werden aus 400 ml Aethanol umkristallisiert. Man erhält 9.2 g (35 mmol) 5 - (9 - Adenyl) - 5 - desoxy - 1.4;3.6 - dianhydro - L - idit. Die eingedampfte Mutterlauge wird in 200 ml Wasser gelöst und kontinuierlich mit ca. 1 Liter einer Mischung von Chloroform/Butanol 9/1 extrahiert. Eindampfen des Extraktes und Umkristallisation aus Chloroform ergibt weitere 3.9 g (15 mmol) Reinprodukt.

Schmp. 202.5—204.5° $[\alpha]_D^{25}$ 22.2 (c 1; Wasser)

Elementaranalyse: $C_{11}H_{13}N_5O_3$ (263.25)

Ber.: C (50.19), H (4.98), N (26.60)

Gef.: C (50.39), H (5.04), N (26.69)

Beispiel Nr. 16:

5-(9-Adenyl)-5-desoxy-1.4;3.6-dianhydro-L-idit:

a) 5-(9-Adenyl)-5-desoxy-1.4;3.6-dianhydro-L-idit-2-methansulfonat:

Die Mischung aus 20.15 g (150 mmol) Adenin-Natriumsalz, 30.2 g (100 mmol) 1.4;3.6 - Dianhydro - D - glucit - 2.5 - dimethan-sulfonat und 300 ml Dimethylsulfoxid wird 24 Stdn. unter Rühren auf 100° erhitzt. Nach dem Abkühlen versetzt man mit 600 ml Wasser, saugt den aus Reaktionsprodukt und nicht umgesetztem Dimethansulfonat bestehenden Niederschlag ab und wäscht zweimal mit je 50 ml Wasser nach. Der Niederschlag wird mit 600 ml Chloroform verrührt, wobei das Dimethansulfonat in Lösung geht und reines Produkt zurückbleibt. Weiteres Produkt erhält man durch zweimalige Extraktion der Chloroformphase mit je 100 ml 2-molarer Salzsäure, Neutralisation der Salzsäurephasen mit verd. Natronlauge und Absaugen des dabei entstehenden Niederschlags. Anschließend kristallisiert man aus Aethanol um und erhält 17.5 g (51.3 mmol) 5 - (9 - Adenyl) - 5 - desoxy - 1.4;3.6 - dianhydro - L - idit - 2 - methansulfonat vom Schmp. 238—240°. $[\alpha]_D^{25}$ 39.8 (c 1; Dimethylformamid).

Elementaranalyse: $C_{12}H_{15}N_5O_5S$ (341.34)

Ber.: C (42.22), H (4.43), N (20.52), S (9.39)

Gef.: C (42.36), H (4.34), N (20.76), S (9.2)

b) 5-(9-Adenyl)-5-desoxy-1.4;3.6-dianhydro-L-idit:

Zur unter Rückfluß siedenden Mischung von 87 g (255 mmol) 5 - (9 - Adenyl) - 5 - desoxy - 1.4;3.6 - dianhydro - L - idit - 2 - methansulfonat und 4 Litern Wasser tropftman mit einer Geschwindigkeit von 10

ml/Stunde die Lösung von 30.6 g (765 mmol) Natriumhydroxid in 250 ml Wasser unter Rühren zu und rührt anschließend noch 5 Stunden unter Rückfluß. Nach dem Abkühlen neutralisiert man die Lösung mit 510 mmol Salzsäure, dampft im Vakuum ein, trocknet den Rückstand azeotrop mit n-Butanol und kocht ihn dreimal mit je 2 Litern n-Butanol aus. Die Butanolextrakte werden im Vakuum eingedampft und aus Aethanol umkristallisiert. Man erhält 41.5 g (158 mmol) 5 - (9 - Adenyl) - 5 - desoxy - 1.4;3.6 - dianhydro - L - idit vom Schmp. 203°. Weitere 10 g (38 mmol) Produkt erhält man durch Eindampfen der Mutterlange, Extraktion des Rückstandes mit Chloroform und Eindampfen des Chloroformextraktes.

Beispiel Nr. 17:
5-(6-Methylmercaptopurin-9-yl)-5-desoxy-1.4;3.6-dianhydro-L-idit:

Die Lösung von 166 g (1 Mol) 6-Methylmercaptopurin und 40 g (1 Mol) Natriumhydroxid in 1 Liter Methanol wird im Vakuum eingedampft und bei 140° bis zur Gewichtskonstanz getrocknet. Man erhält 188 g (1 Mol) des 6-Methylmercaptopurin-9-Natriumsalzes. Diese werden zusammen mit 224 g (1 Mol) 1.4;3.6 - Dianhydro - D - glucit - 5 - methansulfonat in 3 Litern Dimethylformamid suspendiert und 24 Stunden unter Rühren und Stickstoffatmosphäre auf 130° erwärmt. Anschließend destilliert man im Vakuum des Dimethylformamid ab, nimmt den Rückstand in 2 Litern Wasser auf und extrahiert im Rotationsperforator kontinuierlich mit ca. 5 Litern Chloroform. Der Chloroformextrakt wird über wasserfreiem Natriumsulfat geklärt, filtriert und eingedampft. Das verbleibende ölige Reaktionsprodukt erstarrt beim Verreiben mit etwas Wasser zu einem kristallinen Brei, der mit 600 ml Wasser verrührt, abgesaugt und mit 2 mal 75 ml Wasser gewaschen wird. Anschließend trocknet man im Vakuumtrockenschrank bei 120° bis zur Gewichtskonstanz und erhält 147 g (0.5 Mol) 5 - (6 - Methylmercaptopurin - 9 - yl) - 5 - desoxy - 1.4;3.6 - dianhydro - L - idit. Zur Analyse kristallisiert man aus Chloroform/Toluol um.

Schmp. 146—8°; $[\alpha]_D^{25}$ 22.5 (c 1; Chloroform)
Elementaranalyse: $C_{12}H_{14}N_4O_3S$ (294.33)
Ber.: C (48.97), H (4.79), N (19.04), S (10.89)
Gef.: C (48.66), H (4.70), N (18.70), S (10.6)

Beispiel Nr. 18:
5-(6-Methylaminopurin-9-yl)-5-desoxy-1.4;3.6-dianhydro-L-idit:

Das Gemisch aus 8.8 g (30 mmol) 5-(6-Methylmercaptopurin-9-yl)-5-desoxy-1.4;3.6-dianhydro-L-idit, 8.5 ml 33 %iger methanolischer Methylamin-Lösung (90 mmol) und 100 ml Wasser wird bei 130° 20 Stunden im geschlossenen Autoklaven gerührt. Nach dem Abkühlen und Entspannen dampft man zur Trockne ein, löst den Rückstand in 50 ml Wasser und extrahiert kontinuierlich mit Chloroform. Eindampfen des über wasserfreiem Natriumsulfat getrockneten Chloroformextrakts ergibt 7.1 g (25.6 mmol) öliges Rohprodukt, das zur Reinigung in das Hydrochlorid übergeführt und aus Methanol umkristallisiert wird.

Schmp. 247—250° (Z); $[\alpha]_D^{25}$ 35.9 (c 1; Wasser)
Elementaranalyse: $C_{12}H_{15}N_5O_3 \times HCl$ (313.74)
Ber.: C (45.94), H (5.14), N (22.32), Cl (11.30)
Gef.: C (46.08), H (5.25), N (22.29), Cl (11.3)

Beispiel Nr. 19:
5-(6-Dimethylaminopurin-9-yl)-5-desoxy-1.4;3.6-dianhydro-L-idit:

Die Mischung aus 8.8 g (30 mmol) 5-(6-Methylmercaptopurin-9-yl)-5-desoxy-1.4;3.6-dianhydro-L-idit und 50 ml 40 %iger wäßriger Dimethylamin-Lösung wird im geschlossenen Autoklaven 20 Stdn. bei 130° gerührt. Nach dem Abkühlen und Entspannen dampft man im Vakuum ein und kristallisiert aus 20 ml Aethanol um. Man erhält 5.68 g (19.5 mmol) 5 - (6 - Dimethylaminopurin - 9 - yl) - 5 - desoxy - 1.4;3.6 - dianhydro - L - idit. Die analytische Probe wird aus Toluol umkristallisiert.

Schmp. 139—141° $[\alpha]_D^{25}$ 29.5 (c 1; Chloroform)
Elementaranalyse: $C_{13}H_{17}N_5O_3$ (291.31)
Ber.: C (53.60), H (5.88), N (24.04)
Gef.: C (53.90), H (5.97), N (24.11)

Beispiel Nr. 20:
5-(6-Aethylaminopurin-9-yl)-5-desoxy-1.4;3.6-dianhydro-L-idit:

Die Mischung aus 8.8 g (30 mmol) 5-(6-Methylmercaptopurin-9-yl)-5-desoxy-1.4;3.6-dianhydro-L-idit, 8.1 g (100 mmol) Aethylamin-Hydrochlorid, 4.0 g (100 mmol) Natriumhydroxid und 50 ml Wasser wird im geschlossenen Autoklaven 20 Stdn. bei 130° gerührt. Nach dem Abkühlen und Entspannen dampft man zur Trockne ein, löst den Rückstand in 100 ml Wasser und extrahiert 10 mal mit je 100 ml Chloroform. Eindampfen der Chloroformextrakte ergibt 8.15 g öliges Rohprodukt, das mit 14 ml 2-molarer Salzsäure in das Hydrochlorid übergeführt, erneut eingedampft und aus Methanol/Aether umkristallisiert wird. Man erhält 5.7 g (17.4 mmol) 5 - (6 - Aethylaminopurin - 9 - yl) - 5 - desoxy - 1.4;3.6 - dianhydro - L - idit - Hydrochlorid.

Schmp. 240—2°; $[\alpha]_D^{25}$ 37.7 (c 1; Wasser)
Elementaranalyse: $C_{13}H_{17}N_5O_3 \times HCl$ (327.76)
Ber.: C (47.64), H (5.53), N (21.37), Cl (10.82)
Gef.: C (47.78), H (5.61), N (21.40), Cl (10.95)

Beispiel Nr. 21:

5-(6-Pyrrolidinopurin-9-yl)-5-desoxy-1.4;3.6-dianhydro-L-idit:

Das Gemisch aus 8.8 g (30 mmol) 5-(6-Methylmercaptopurin-9-yl)-5-desoxy-1.4;3.6-dianhydro-L-idit und 25 ml (300 mmol) Pyrrolidin wird 20 Stdn. im geschlossenen Autoklaven bei 130° gerührt. Nach dem Abkühlen und Entspannen destilliert man überschüssiges Pyrrolidin ab, gegen Ende unter Zugabe von Wasser, und kristallisiert den Rückstand aus Wasser um. Man erhält 8.43 g (26.6 mmol) 5 - (6 - Pyrrolidinopurin - 9 - yl) - 5 - desoxy - 1.4;3.6 - dianhydro - L - idit.

Schmp. 183.5—185.5°; $[\alpha]_D^{25}$ 34.5 (c 1; Chloroform)

Elementaranalyse: $C_{15}H_{19}N_5O_3$ (317.35)

Ber.: C (56.77), H (6.03), N (22.07)

Gef.: C (57.00), H (6.07), N (22.08)

Beispiel Nr. 22:

5-(6-Piperidinopurin-9-yl)-5-desoxy-1.4;3.6-dianhydro-L-idit:

Analog zum vorgehenden Beispiel werden 8.8 g (30 mmol) 5-(6-Methylmercaptopurin-9-yl)-5-desoxy-1.4;3.6-dianhydro-L-idit zusammen mit 25 ml (253 mmol) Piperidin 20 Stunden bei 130° im geschlossenen Autoklaven gerührt. Nach Abdestillieren des überschüssigen Piperidins (gegen Ende unter Zugabe von Wasser) verreibt man den öligen Rückstand mit Toluol. Das nun feste Rohprodukt wird abgesaugt, getrocknet und aus Wasser umkristallisiert. Man erhält 7.7 g (23.2 mmol) reinen 5 - (6 - Piperidinopurin - 9 - yl) - 5 - desoxy - 1.4;3.6 - dianhydro - L - idit.

Schmp. 136—139°; $[\alpha]_D^{25}$ 30.1 (c 1; Chloroform)

Elementaranalyse: $C_{16}H_{21}N_5O_3$ (331.38)

Ber.: C (57.99), H (6.39), N (21.13)

Gef.: C (58.14), H (6.48), N (21.13)

Beispiel Nr. 23:

5-(6-Morpholinopurin-9-yl)-5-desoxy-1.4;3.6-dianhydro-L-idit:

Analog zu den voranstehenden Beispielen 21 und 22 erhält man aus 8.8 g (30 mmol) 5-(6-Methylmercaptopurin-9-yl)-5-desoxy-1.4;3.6-dianhydro-L-idit und 25 ml (287 mmol) Morpholin nach 20 Stdn. Rühren bei 130° im geschlossenen Autoklaven, Abdestillieren des überschüssigen Morpholins unter Wasserzugabe und Umkristallisation des Rückstandes aus Wasser 6.9 g (18.7 mmol) reinen 5 - (6 - Morpholinopurin - 9 - yl) - 5 - desoxy - 1.4;3.6 - dianhydro - L - idit in Form des Dihydrats.

Schmp. 84—88°; $[\alpha]_D^{25}$ 26.4 (c 1; Chloroform)

Elementaranalyse: $C_{15}H_{19}N_5O_4 \times 2\ H_2O$ (369.38)

Ber.: C (48.78), H (6.28), N (18.96)

Gef.: C (48.71), H (6.31), N (18.74)

Beispiel Nr. 24:

5-[6-(4-Metehylpiperazino)-purin-9-yl]-5-desoxy-1.4;3.6-dianhydro-L-idit:

Analog zu den vorhergehenden Beispielen erhält man aus 8.8 g (30 mmol) 5-(6-Methylmercaptopurin-9-yl)-5-desoxy-1.4;3.6-dianhydro-L-idit und 25 ml (225 mmol) N-Methylpiperazin 10.2 g (29.4 mmol) Rohprodukt. Umkristallisation aus Toluol ergibt 6.3 g (18.2 mmol) Reinprodukt.

Schmp. 166—7°; $[\alpha]_D^{25}$ 31.1 (c 1; Chloroform)

Elementaranalyse: $C_{16}H_{22}N_6O_3$ (346.40)

Ber.: C (55.48), H (6.40), N (24.26)

Gef.: C (55.60), H (6.45), N (23.91)

Beispiel Nr. 25:

5-(6-Benzylaminopurin-9-yl)-5-desoxy-1.4;3.6-dianhydro-L-idit:

Das Gemisch aus 8.8 g (30 mmol) 5-(6-Methylmercaptopurin-9-yl)-5-desoxy-1.4;3.6-dianhydro-L-idit und 25 ml (229 mmol) Benzylamin wird im geschlossenen Autoklaven 20 Stdn. bei 130° gerührt. Nach dem Abkühlen und Entspannen destilliert man das überschüssige Benzylamin—gegen Ende unter Zugabe von Wasser—im Vakuum ab, kristallisiert den öligen Rückstand aus Aceton um und erhält 6.9 g (19.5 mmol) 5 - (6 - Benzylamino - purin - 9 - yl) - 5 - desoxy - 1.4;3.6 - dianhydro - L - idit. Die analytische Probe wird nochmals aus Toluol umkristallisiert.

Schmp. 151—3°; $[\alpha]_D^{25}$ 28.7 (c 1; Chloroform)

Elementaranalyse: $C_{18}H_{19}N_5O_3$ (353.38)

Ber.: C (61.18), H (5.42), N (19.82)

Gef.: C (61.17), H (5.44), N (19.82)

Beispiel Nr. 26:

5-[6-(3-Phenylpropyl)-aminopurin-9-yl]-5-desoxy-1.4;3.6-dianhydro-L-idit:

Das Gemisch aus 19.1 g (65 mmol) 5-(6-Methylmercaptopurin-9-yl)-5-desoxy-1.4;3.6-dianhydro-L-idit und 94 ml (650 mmol) 3-Phenylpropylamin wird im geschlossenen Stahlautoklaven 36 Stdn. auf 150°

erhitzt. Nach dem Abkühlen und Entspannen gibt man 80 ml Essigsäure und 500 ml Wasser zu und extrahiert zweimal mit je 300 ml Chloroform. Die Chloroformextrakte werden mit 300 ml Wasser gewaschen und zweimal mit der Lösung von 25 ml 37 %iger Salzsäure in 200 ml Wasser extrahiert. Die salzsauren Extrakte werden mit Natronlauge alkalisiert und mit 400 ml Dichlormethan extrahiert. Der Dichlormethanextrakt ergibt nach dem Trocknen über wasserfreiem Natriumsulfat, Filtrieren und Eindampfen 20 g (52.4 mmol) Rohbase, die in 150 ml Isopropanol in der Wärme gelöst und mit 15 ml 37 %iger Salzsäure (180 mmol) versetzt werden. Beim Abkühlen kristallisieren 18.8 g (41.4 mmol) reines Produkt in Form des Dihydrochloride aus.

Schmp. 159—62°; $[\alpha]_D^{25}$ 30.6 (c 0.4; Wasser)

Elementaranalyse: $C_{20}H_{23}N_5O_3 \times 2$ HCl (454.36)

Ber.: C (52.86), H (5.55), N (15.41), Cl (15.61)

Gef.: C (53.23), H (5.55), N (15.41), Cl (14.4)

Beispiel Nr. 27:

5-[6-(2-Phenyläthyl)-aminopurin-9-yl]-5-desoxy-1.4;3.6-dianhydro-L-idit:

Herstellung analog Beispiel 26 durch Umsetzung von 5-(6-Methylmercaptopurin-9-yl)-5-desoxy-1.4;3.6-dianhydro-L-idit mit überschüssigem 2-Phenyläthylamin bei 130°. Isolierung als Hydrochlorid. Ausbeute nach Umkristallisation aus Aethanol: 58:

Schmp. 214—18°; $[\alpha]_D^{25}$ 32.7 (c 0.39; Wasser)

Elementaranalyse: $C_{19}H_{21}N_5O_3 \times HCl$ (403.87)

Ber.: C (56.51), H (5.49), N (17.34), Cl (8.78)

Gef.: C (56.42), H (5.87), N (17.39), Cl (9.1)

Beispiel Nr. 28:

5-[6-(4-Phenylbutyl)-aminopurin-9-yl)-5-desoxy-1.4;3.6-dianhydro-L-idit:

Herstellung analog Beispiel 26 durch Umsetzung von 5-(6-Methylmercaptopurin-9-yl)-5-desoxy-1.4;3.6-dianhydro-L-idit mit überschüssigem 4-Phenylbutylamin bei 150°. Isolierung als Hydrochlorid. Ausbeute nach Umkristallisation aus Isopropanol: 47%

Schmp. 172—6°; $[\alpha]_D^{25}$ 30.6 (c 0.4; Wasser).

Elementaranalyse: $C_{21}H_{25}N_5O_3 \times HCl$ (431.92)

Ber.: (58.40), H (6.07), N (16.21), Cl (8.21)

Gef.: C (58.15), H (6.15), N (15.94), Cl (8.5)

Beispiel Nr. 29:

2-(9-Adenyl)-2-desoxy-1.4;3.6-dianhydro-D-glucit:

Die Mischung aus 112 g (0.5 Mol) 1.4;3.6-Dianhydro-D-mannit-2-methansulfonat (hergestellt nach Beispiel 4 a), 86 g (0.55 Mol) Adenin-Natriumsalz und 1500 ml wasserfr. Dimethylsulfoxid wird 24 Stdn. unter Stickstoffatmosphäre bei 120° gerührt. Unter reduziertem Druck destilliert man das Dimethylsulfoxid ab, löst den Rückstand in 1 Liter Wasser, extrahiert lipophile Nebenprodukte 2 mal mit je 300 ml Chloroform und konzentriert die Wasserphase auf ca. 200 ml. Das auskristallisierende Rohprodukt ergibt nach Umkristallisation aus Aethanol/Wasser 47.4 g (0.18 Mol) 2 - (9 - Adenyl) - 2 - desoxy - 1.4;3.6 - dianhydro - D - glucit.

Schmp. 265—7°; $[\alpha]_D^{25}$ 20.8 (c 1.0; Wasser).

Elementaranalyse: $C_{11}H_{13}N_5O_3$ (263.25)

Ber.: C (50.19), H (4.98), N (26.60)

Gef.: C (49.97), H (5.04), N (26.80)

Beispeil Nr. 30:

5-(7-Theophyllinyl)-5-desoxy-1.4;3.6-dianhydro-L-idit:

Die Mischung aus 44.8 g (0.2 Mol) 1.4;3.6-Dianhydro-D-glucit-5-methansulfonat, 80 g (0.4 Mol) Theophyllin-Natriumsalz und 600 ml Dimethylsulfoxid wird 48 Stdn. unter Stickstoffatmosphäre bei 130° gerührt. Man destilliert das Dimethylsulfoxid im Vakuum ab, löst den Rückstand in 400 ml heißem Wasser, extrahiert nach dem Abkühlen zweimal mit je 200 ml Chloroform und schüttelt die Chloroformextrakte mit 200 ml Wasser aus. Die vereinigten Wasserphasen werden mit 30 g Aktivkohle aufgekocht, filtriert und das Filtrat nach Neutralisation zur Trockne eingedampft. Der Rückstand wird mit 1 Liter Aethanol gerührt, filtriert und ernent eingedampft. Zur Entfernung restlichen Theophyllins löst man dann in 700 ml 2 %iger Natronlauge und extrahiert das Reaktionsprodukt kontinuierlich mit Chloroform. Der Chloroformextrakt ergibt nach dem Trocknen über wasserfreiem Natriumsulfat und Eindampfen 27.5 g (89 mmol) 5 - (7 - Theophyllinyl) - 5 - desoxy - 1.4;3.6 - dianhydro - L - idit. Die analytische Probe wird aus Aceton/Aethanol umkristallisiert.

Schmp. 196—8°; $[\alpha]_D^{25}$ −27.9 (c 0.5; Wasser)

Elementaranalyse: $C_{13}H_{16}N_4O_5$ (308.30)

Ber.: C (50.65), H (5.23), N (18.17)

Gef.: C (50.68), H (5.32), N (18.06)

Beispiel Nr. 31:

5-(2-Theophyllin-7-yläthylamino)-5-desoxy-1.4;3.6-dianhydro-L-idit:

Die Mischung aus 8.6 g (30 mmol) 7-(2-Bromäthyl)-theophyllin, 8 ml Aethanol und 10.8 g (75 mmol) 5 - Amino - 5 - desoxy - 1.4;3.6 - dianhydro - L - idit (Herstellung s. Beispiele 1 und 2) wird 24 Stdn. unter Rückfluß gekocht und dann unter reduziertem Druck eingedampft. Der Rückstand wird in 100 ml Wasser gelöst und 10 mal mit je 100 ml Chloroform extrahiert, wobei der erste Chloroformextrakt verworfen wird. Die Chloroformextrakte 2—10 werden unter reduziertem Druck eingedampft. Ueberführung des Rückstandes in das Hydrochlorid und Umkristallisation aus Methanol ergeben 7.93 g (20.4 mmol) 5 - (2 - Theophyllin - 7 - yläthylamino) - 5 - desoxy - 1.4;3.6 - dianhydro - L - idit - Hydrochlorid.

Schmp. 216—221°; $[\alpha]_D^{25}$ 34.1 (c 0.39; Wasser)

Elementaranalyse: $C_{15}H_{21}N_5O_5 \times HCl$ (387.83)

Ber.: C (46.46), H (5.72), N (18.06), Cl (9.14)

Gef.: C (46.25), H (5.99), N (17.46), Cl (9.5)

Beispiel Nr. 32:

5-(3-Theophyllin-7-ylpropylamino)-5-desoxy-1.4;3.6-dianhydro-L-idit:

Zur unter Rückfluß siedenen Lösung von 109 g (0.75 Mol) 5-Amino-5-desoxy-1.4;3.6-dianhydro-L-idit in 1 Liter Aethanol gibt man über einen Soxhlet Extraktoraufsatz innerhalb 10 Stdn. 75.3 g (0.25 Mol) 7 - (3 - Brompropyl) - theophyllin zu, kocht 48 Stdn. nach und dampft unter reduziertem Druck ein. Der Rückstand wird in 1.5 Liter Wasser gelöst, mit Salzsäure auf pH=4 gebracht, mit 500 ml Chloroform gewaschen, dann mit Natriumhydroxid auf pH=8.5 eingestellt und im Rotationsperforator (Normag) kontinuierlich mit Chloroform extrahiert, bis sämtliches Reaktionsprodukt in die Chloroformphase übergegangen ist. Der Chloroformextrakt wird unter reduziertem Druck eingedampft, in Isopropanol gelöst, durch Zugabe von Methansulfonsäure in das Hydrogenmethansulfonat übergeführt und nach dem Eindampfen aus Aethanol/Isopropanol umkristallisiert. Man erhält 83.5 g (0.18 Mol) 5 - (3 - Theophyllin - 7 - yl - propylamino) - 5 - desoxy - 1.4;3.6 - dianhydro - L - idit - Hydrogenmethansulfonat.

Schmp. 145—147°; $[\alpha]_D^{25}$ 24.2 (c 0.5; Wasser)

Elementaranalyse: $C_{16}H_{23}N_5O_5 \times CH_3SO_3H$ (461.50)

Ber.: C (44.24), H (5.90), N (15.17), S (6.95)

Gef.: C (44.11), H (5.92), N (15.03), S (7.2)

Die Wasserphase der Perforation wird unter reduziertem Druck eingedampft, mit Aethanol extrahiert, filtriert und eingedampft, der Eindampfrückstand mit Chloroform extrahiert, filtriert und erneut eingedampft. Dieser Rückstand ergibt nach Extraktion mit n-Butanol, Filtrieren, Eindampfen und Trocknen im Vakuumtrockenschrank bei 80°, 59 g (406 mmol=81%) des überschüssigen 5-Amino-5-desoxy-1.4;3.6-dianhydro-L-idits zurück.

Beispiel Nr. 33:

5-(4-Theophyllin-7-ylbutylamino)-5-desoxy-1.4;3.6-dianhydro-L-idit:

Herstellung analog Beispiel 32 durch Umsetzung von 7-(4-Brombutyl)-theophyllin mit überschüssigem 5 - Amino - 5 - desoxy - 1.4;3.6 - dianhydro - L - idit unter Zusatz von Kaliumjodid. Isolierung als Hydrochlorid. Ausbeute nach Umkristallisation aus Isopropanol/Chloroform: 44%.

Schmp. 196—8°; $[\alpha]_D^{25}$ 29.8 (c 0.41; Wasser)

Elementaranalyse: $C_{17}H_{25}N_5O_5 \times HCl$ (415.88)

Ber.: C (49.10), H (6.30), N (16.84), Cl (8.52)

Gef.: C (48.77), H (6.56), N (16.57), Cl (8.6)

Beispiel Nr. 34:

5-(5-Theophyllin-7-ylpentylamino)-5-desoxy-1.4;3.6-dianhydro-L-idit:

Herstellung analog Beispiel 33 durch Umsetzung von 7-(5-Brompentyl)-theophyllin mit überschüssigem 5 - Amino - 5 - desoxy - 1.4;3.6 - dianhydro - L - idit. Isolierung als Hydrochlorid. Ausbeute nach Umkristallisation aus Aethanol/Chloroform: 69%.

Schmp. 230—233°; $[\alpha]_D^{25}$ 28.9 (c 0.4; Wasser)

Elementaranalyse: $C_{18}H_{27}N_5O_5 \times HCl$ (429.90)

Ber.: C (50.29), H (6.56), N (16.29), Cl (8.25)

Gef.: C (50.40), H (6.69), N (16.38), Cl (8.3)

Beispiel Nr. 35:

5-(6-Theophyllin-7-ylhexylamino)-5-desoxy-1.4;3.6-dianhydro-L-idit:

Herstellung analog Beispiel 33 durch Umsetzung von 7-(6-Bromhexyl)-theophyllin mit überschüssigem 5 - Amino - 5 - desoxy - 1.4;3.6 - dianhydro - L - idit. Isolierung als Hydrochlorid. Ausbeute nach Umkristallisation aus Chloroform/Aceton: 39%.

Schmp. 180—183°; $[\alpha]_D^{25}$ 27.1 (c 0.4; Wasser)

Elementaranalyse: $C_{19}H_{29}N_5O_5 \times HCl$ (443.93)

Ber.: C (51.41), H (6.81), N (15.78), Cl (7.99)

Gef.: C (51.12), H (6.90), N (15.59), Cl (8.6)

Beispiel Nr. 36:

5-(4-Theophyllin-7-yl-2-butylamino)-5-desoxy-1.4;3.6-dianhydro-L-idit:

Zur Lösung von 10 g (40 mmol) 7-(3-Oxobutyl)-theophyllin in 75 ml Aethanol gibt man die Lösung von 5.8 g (40 mmol) 5 - Amino - 5 - desoxy - 1.4;3.6 - dianhydro - L - idit in 40 ml Methanol, setzt die Suspension von 1.5 g 10 %igem Pd/C-Katalysator in 100 ml Aethanol zu, spült mit Stickstoff und hydriert im geschlossenen Stahlautoklaven unter 50 atm Wasserstoffdruck 20 Stdn. bei Raumtemperatur und 8 Stdn. bei 50°, wobei ca, 250-mal/min. geschüttelt wird. Nach dem Abkühlen, Entspannen und Abfiltrieren des Katalysators dampft man das Filtrat unter reduz. Druck ein, stellt die Lösung des Rückstands in Wasser mit Salzsäure auf pH=2 ein und perforiert 8 Stdn. im Rotationsperforator mit Chloroform, um Nebenprodukte zu extrahieren. Man stellt die wäßrige Phase auf pH=4 und perforiert erneut mit Chloroform. Beide Chloroformphasen werden verworfen. Nach Einstellung auf pH=7 perforiert man 8 Stdn. mit Chloroform. Aus dieser Chloroformphase erhält man nach dem Trocknen über wasserfreiem Natriumsulfat und Eindampfen unter reduziertem Druck 8.5 g (22.4 mmol) 5 - (4 - Theophyllin - 7 - yl - 2 - butylamino) - 5 - desoxy - 1.4;3.6 - dianhydro - L - idit(Gemisch der beiden Epimeren) in Form eines farblosen Oels. Zur Analyse überführt man in das Hydrochlorid und fällt aus Isopropanol mit Pentan um.

Schmp. 155—166° (Z); $[\alpha]_D^{25}$ 6.7 (c 0.39; Wasser).

Elementaranalyse: $C_{17}H_{25}N_5O_5 \times HCl$ (415.88)

Ber.: C (49.10), H (6.30), N (16.84), Cl (8.52)

Gef.: C (49.11), H (6.48), N (16.28), Cl (8.5)

Beispiel Nr. 37:

5-Theophyllin-7-yl-5-desoxy-1.4;3.6-dianhydro-L-idit-2-methansulfonat:

Das Gemisch aus 36 g (178 mmol) Theophyllin Natrium, 36 g (120 mmol) 1.4;3.6-Dianhydro-D-glucit-2.5-dimethansulfonat und 500 ml wasserfr. Diäthylenglykoldiäthyläther wird 2 Tage unter Feuchtigkeitsausschluß bei 180° gerührt. Man filtriert, engt das Filtrat unter reduziertem Druck ein, versetzt den Rückstand mit 800 ml Wasser, filtriert und extrahiert das Filtrat 3 mal mit je 600 ml Chloroform. Die Chloroformphasen werden nach dem Waschen mit 200 ml 2-molarer Natronlauge und Trocknen über wasserfr. Natriumsulfat auf ca. 200 ml Volumen konzentriert und das Rohprodukt durch Zugabe von Petroläther gefällt. Die Fällung wird in siedendem Chloroform gelöst. Beim Abkühlen kristallisiert nicht umgesetztes 1.4;3.6 - Dianhydro - D - glucit - 2.5 - dimethansulfonat aus und wird abgesaugt. Aus dem Filtrat fällt man durch erneute Zugabe von Petroläther 24.3 g (63 mmol) 5 - Theophyllin - 7 - yl - 5 - desoxy - 1.4;3.6 - dianhydro - L - idit - 2 - methansulfonat aus, das nach Umkristallisation aus Aethanol den Schmp. 191—4° besitzt. $[\alpha]_D^{25}$ 17.2 (c 0.51; Dimethylformamid)

Elementaranalyse: $C_{14}H_{18}N_4O_7S$ (386.39)

Ber.: C (43.53), H (4.69), N (14.50), S (8.30)

Gef.: C (43.47), H (4.78), N (14.02), S (8.30)

Beispiel Nr. 38:

5-[6-(3-p-Chlorphenylpropyl)-aminopurin-9-yl]-5-desoxy-1.4;3.6-dianhydro-L-idit:

Herstellung analog Beispiel 26 durch Umsetzung von 5-(6-Methylmercaptopurin-9-yl)-5-desoxy-1.4;3.6-dianhydro-L-idit mit überschüssigem 3 - (4 - Chlorphenyl) - propylamin in Aethanol bei 150° im geschlossenen Stahlautoklaven. Isolierung als Hydrochlorid mit 1/2 Mol Kristallwasser.

Schmp. nach Umkristallisation aus Aethanol 116—120° $[\alpha]_D^{25}$ 22.5° (c 0.2; Wasser)

Elementaranalyse: $C_{20}H_{22}ClN_5O_3 \times HCl \times 0.5\ H_2O$ (461.35)

Ber.: C (52.07), H (5.24), N (15.18), Cl (15.37)

Gef.: C (52.34), H (5.20), N (15.15), Cl (15.50)

Beispiel Nr. 39:

2-(7-Theophyllinyl)-2-desoxy-1.4;3.6-dianhydro-D-glucit:

Herstellung analog zu Beispiel Nr. 30 durch Umsetzung von 1.4;3.6-Dianhydro-D-mannit-2-methansulfonat mit Theophyllin-Kalium in wasserfr. Dimethylsulfoxid bei 120°C. Nach Umkristallisation aus Methanol/Wasser erhält man reinen 2 - (7 - Theophyllinyl) - 2 - desoxy - 1.4;3.6 - dianhydro - D - glucit mit Schmp. 230—31°C und $[\alpha]_D^{25}$ −27.3 (c 0.4; Wasser).

Elementaranalyse: $C_{13}H_{16}N_4O_5$ (308.30)

Ber.: C (50.65), H (5.23), N (18.17)

Gef.: C (50.69), H (5.26), N (17.96)

Beispiel Nr. 40:

2-(7-Theophyllinyl)-2-desoxy-1.4;3.6-dianhydro-D-glucit-5-methansulfonat:

Die Mischung aus 150 g (0.5 Mol) 1.4;3.6-Dianhydro-D-mannit-2.5-dimethansulfonat, 87 g (0.4 Mol) Theophyllin-Kalium und 1500 ml wasserfr. Dimethylsulfoxid wird 68 Std. bei 110°C im geschlossenen Stahlautoklaven unter Stickstoffatmosphäre gerührt. Nach weitgehender Abdestillation des Dimethylsulfoxids unter reduziertem Druck verrührt man den Rückstand mit 500 ml Wasser und 200 ml Chloroform,

saugt den Niederschlag (N 1) ab, trennt aus dem Filtrat die Chloroformphase (C) ab und verwirft die Wasserphase.

N 1 wird mit 400 ml Wasser verrührt, abgesaugt, getrocknet, mit 200 ml Dichlormethan aufgekocht, nach dem Abkühlen 2 Stdn, stehen gelassen und erneut abgesaugt. Man erhält fast reines Produkt (N 2).

Die Chloroformlösung C wird eingedampft und mit 200 ml Aceton aufgekocht. Das nach Abkühlen ausfallende Disubstitutionsprodukt wird abgetrennt, das Filtrat wird eingedampft und aus Dichlormethan umkristallisiert. Das Kristallisat (N 3) wird mit N 2 vereinigt und aus Aceton umkristallisiert. Man erhält 44.6 g (115 mmol) reines 2 - (7 - Theophyllinyl) - 2 - desoxy - 1.4;3.6 - dianhydro - D - glucit - 5 - methansulfonat mit Schmp. 200—203°C und $[\alpha]_D^{25}$ 31.3 (c 0.40; Dimethylformamid).

Elementaranalyse: $C_{14}H_{18}N_4O_7S$ (386.39)

Ber.: C (43.52), H (4.69), N (14.50), S (8.30)

Gef.: C (43.29), H (4.81), N (14.07), S (8.2)

Umkristallisation des Disubstitutionsproduktes aus 96 %igem Aethanol ergibt reisnen 2.5 - Bis - (7 - theophyllinyl) - 2.5 - didesoxy - 1.4;3.6 - dianhydro - L - idit in Form des Halbhydrates mit Schmp. 303—305° (Z) und $[\alpha]_D^{20}$ −61.5 (c 0.4; Dimethylformamid)

Elementaranalyse: $C_{20}H_{22}N_8O_6 \times 0.5\ H_2O$ (479.45)

Ber.: C (50.10), H (4.83), N (23.37)

Gef.: C (50.36), H (4.61), N (23.28)

Beispiel Nr. 41:

2-(7-Theophyllinyl)-2-desoxy-1.4;3.6-dianhydro-D-glucit-5-p-toluolsulfonat:

Herstellung analog Beispiel 40 durch Umsetzung von 1.4;3.6 - Dianhydro - D - mannit - 2.5 - di - p - toluolsulfonat mit Theophyllin-Kalium in Dimethylsulfoxid. Umkristallisation aus Chloroform/Aethanol ergibt reines Produkt mit Schmp. 175—176° und $[\alpha]_D^{20}$ 37.3 (c 0.4; Dimethylformamid).

Elementaranalyse: $C_{20}H_{22}N_4O_7S$ (462.49)

Ber.: C (51.94), H (4.80), N (12.11), S (6.93)

Gef.: C (52.07), H (5.02), N (12.20), S (7.0)

Beispiel Nr. 42

2-Dimethylamino-2-desoxy-1.4;3.6-dianhydro-D-glucit:

Die Mischung aus 45 g (0.2 Mol) 1.4;3.6-Dianhydro-D-mannit-2-methansulfonat und 150 ml 40 %iger wäßriger Dimethylamin-Lösung wird 18 Stdn. unter Stickstoffatmosphäre im geschlossenen Stahlautoklaven bei 150°C gerührt. Nach dem Abkühlen und Entspannen gibt man 8.0 g (0.2 Mol) Natriumhydroxid und 10 g Aktivkohle zu, kocht 15 Min. unter Rückfluß, filtriert, dampft das Filtrat ein und trocknet aceotrop mit Toluol. Der Rückstand wird mit Chloroform extrahiert. Eindampfen des Extraktes ergibt 25 g (144 mmol) langsam erstarrendes Rohprodukt. Zur Reinigung überführt man in das Hydrochlorid und kristallisiert zweimal aus Isopropanol um. Man erhält 2 - Dimethylamino - 2 - desoxy - 1.4;3.6 - dianhydro - D - glucit - Hydrochlorid mit Schmp. 175°C und $[\alpha]_D^{20}$ 59.2 (c 0.5; Wasser).

Elementaranalyse: $C_8H_{15}NO_3 \times HCl$ (209.68)

Ber.: C (45.82), H (7.69), N (6.68), Cl (16.91)

Gef.: C (45.72), H (8.03), N (6.28), Cl (17.0).

**Patentansprüche**

1. Amino-desoxy-1.4;3.6-dianhydro-hexit-Derivate der allgemeinen Formel I,

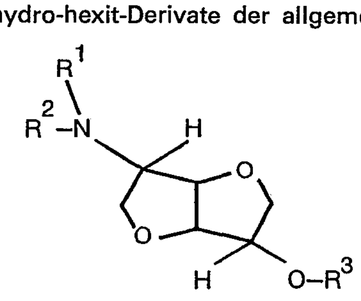

(I)

worin $R^1$ und $R^2$ jeweils unabhängig voneinander ein Wasserstoffatom oder eine Niedrigalkylgruppe mit 1 bis 4 C-Atomen

oder worin $R^1$ ein Wasserstoffatom und $R^2$ einen Adamant-(1)yl-Rest

oder worin $R^1$ und $R^2$ gemeinsam mit dem Stickstoffatom, an das sie gebunden sind, (a) den Rest eines cyclischen, nicht aromatischen, gegebenenfalls ein weiteres Heteroatom enthaltenden sekundären Amins, das ausgewählt ist aus der Gruppe Pyrrolidin, Piperidin, Morpholin, Methylpiperazin, oder (b) den Aden(9)yl-Rest, den 6-Alkylamino-purin(9)yl- oder 6-Dialkylamino-purin(9)yl-Rest, wobei die Alkylgruppen 1 bis 4 C-Atome aufweisen, den gegebenenfalls halogensubstituierten 6-(ω-Phenylalkyl)-amino-purin(9)yl-Rest, dessen Alkylgruppe 1 bis 8 C-Atome aufweist, oder den 6-Pyrrolidinopurin(9)yl-, 6-Piperidinopurin(9)yl-, 6-Morpholinopurin(9)yl- oder 6-Methylpiperazinopurin(9)yl-Rest oder (c) den 6-Alkylmercaptopurin(9)-yl-Rest oder (d) den Theophyllin(7)yl-Rest oder (e) den 6-Chlorpurin-9-yl-Rest

oder worin $R^1$ ein Wasserstoffatom oder eine Niedrigalkylgruppe mit 1 bis 4 C-Atomen und $R^2$ einem ω-Theophyllin(7)yl-alkyl-Rest oder einen ω-Theobromin-1-yl-alkyl-Rest oder einen ω-(N,N'-di-niedrigalkyl-substituierten Xanthin-N''-yl)-alkyl-Rest, wobei "niedrigalkyl" eine Alkylgruppe mit 1 bis 5 C-Atomen bedeutet, oder einen ω-Adenin-9-yl-alkyl-Rest, wobei der Alkylrest 2 bis 7 C-Atome aufweisen und geradkettig oder verzweigt sein kann,

und worin $R^3$ ein Wasserstoffatom, eine Methansulfonyl- oder Toluolsulfonylgruppe bedeuten, sowie deren Säureadditionssalze.

2. 5-Amino-5-desoxy-1.4;3.6-dianhydro-L-idit-Derivate der allgemeinen Formel V,

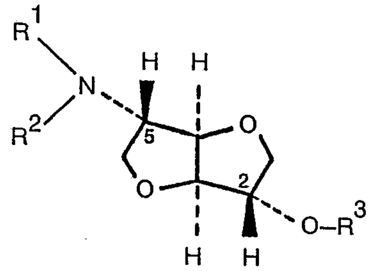

(V)

worin $R^1$, $R^2$ und $R^3$ die in Anspruch 1 genannten Bedeutungen besitzen, sowie deren Säureadditionssalze.

3. 5-Amino-5-desoxy-1.4;3.6-dianhydro-D-glucit-Derivate der allgemeinen Formel VI,

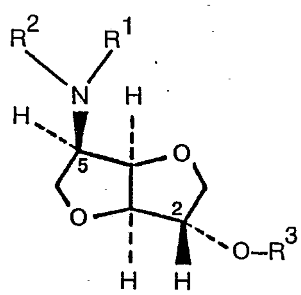

(VI)

worin $R^1$, $R^2$ und $R^3$ die in Anspruch 1 genannten Bedeutungen besitzen, sowie deren Säureadditionssalze.

4. 2-Amino-2-desoxy-1.4;3.6-dianhydro-D-glucit-Derivate der allgemeinen Formel VII,

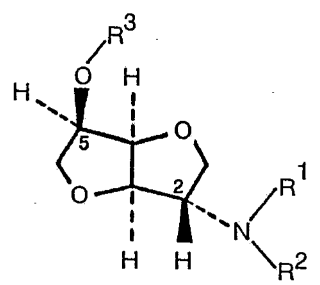

(VII)

worin $R^1$, $R^2$ und $R^3$ die in Anspruch 1 genannten Bedeutungen besitzen, sowie deren Säureadditionssalze.

5. 2-Amino-2-desoxy-1.4;3.6-dianhydro-D-mannit-Derivate der allgemeinen Formel VIII,

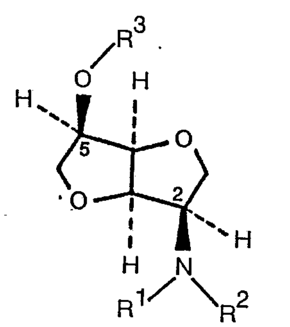

(VIII)

worin $R^1$, $R^2$ und $R^3$ die in Anspruch 1 genannten Bedeutungen besitzen, sowie deren Säureadditionssalze.

6. (Purin-9-yl)-desoxy-1.4;3.6-dianhydro-hexit-Derivate der allgemeinen Formel IX,

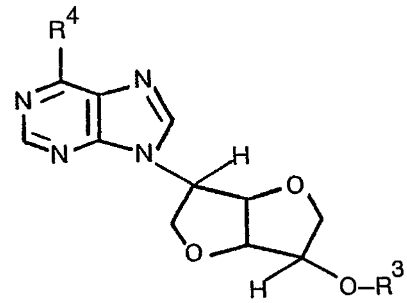

(IX)

worin $R^3$ ein Wasserstoffatom, eine Methansulfonyl- oder Toluolsulfonylgruppe und $R^4$ eine Aminogruppe, eine Alkylmercapto-, Alkylamino- oder Dialkylaminogruppe mit 1 bis 4 C-Atomen, eine ω-Phenyl-alkylaminogruppe, deren Alkylrest 1 bis 8 C-Atome aufweist und deren Phenylrest gegebenenfalls halogensubstituiert ist, oder den Rest eines cyclischen, nicht-aromatischen, gegebenenfalls ein weiteres Heteroatom enthaltenden sekundaren Amins das ausgewählt ist aus der Gruppe Pyrrolidin, Piperidin, Morpholin, Methylpiperazin, oder eine Chloratom bedeuten, sowie deren Säureadditionssalze.

7. 5-(Purin-9-yl)-5-desoxy-1.4;3.6-dianhydro-L-idit-Derivate der allgemeinen Formel X,

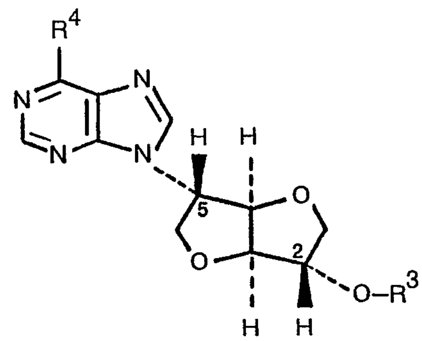

(X)

worin $R^3$ und $R^4$ die in Anspruch 6 genannten Bedeutungen besitzen, sowie deren Säureadditionssalze.

8. 5-(Purin-9-yl)-5-desoxy-1.4;3.6-dianhydro-D-glucit-Derivate der allgemeinen Formel XI,

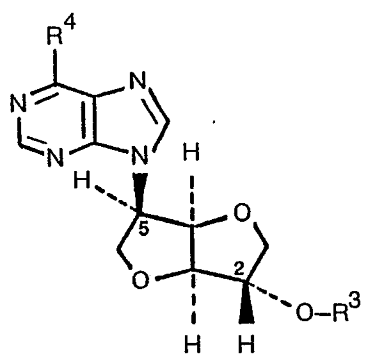

(XI)

worin $R^3$ und $R^4$ die in Anspruch 6 genannten Bedeutungen besitzen, sowie deren Säureadditionssalze.

9. 2-(Purin-9-yl)-2-desoxy-1.4;3.6-dianhydro-D-glucit-Derivate der allgemeinen Formel XII,

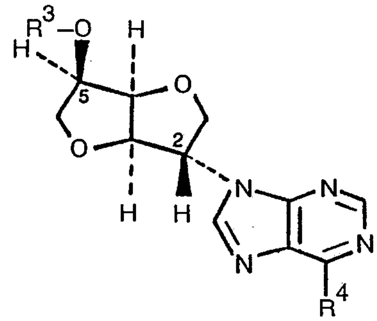

(XII)

worin $R^3$ und $R^4$ die in Anspruch 6 genannten Bedeutungen besitzen, sowie deren Säureadditionssalze.

10. 2-(Purin-9-yl)-2-desoxy-1.4;3.6-dianhydro-D-mannit-Derivate der allgemeinen Formel XIII,

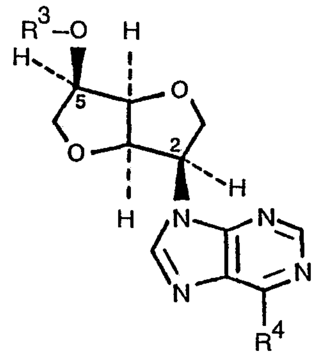

(XIII)

worin $R^3$ und $R^4$ die in Anspruch 6 genannten Bedeutungen besitzen, sowie deren Säureadditionssalze.

11. (Theophyllin-7-yl)-desoxy-1.4;3.6-dianhydro-hexit-Derivate der allgemeinen Formel XIV,

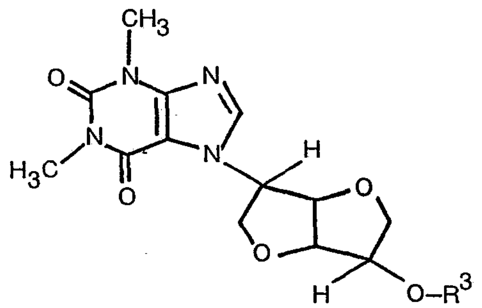

(XIV)

worin $R^3$ ein Wasserstoffatom, eine Methansulfonyl- oder Toluolsulfonylgruppe bedeutet, sowie deren Säureadditionssalze.

12. (ω-Purinylalkylamino)-desoxy-1.4;3.6-dianhydro-hexit-Derivate der allgemeinen Formel XV,

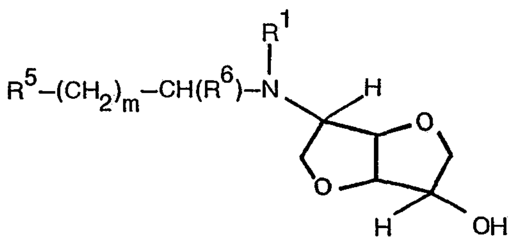

(XV)

worin $R^1$ ein Wasserstoffatom oder eine Niedrigalkylgruppe mit 1 bis 4 C-Atomen,

$R^5$ den Adenin-9-yl-Rest, den Theophyllin-7-yl-Rest, den Theobromin-1-yl-Rest oder einen weiteren N,N' - diniedrigalkylxanthin - N'' - yl - Rest, wobei "niedrigalkyl" eine Alkylgruppe mit 1 bis 5 C-Atomen bedeutet,

$R^6$ ein Wasserstoffatom oder eine Methylgruppe und m eine ganze Zahl von 1 bis 6 bedeuten, sowie deren Säureadditionssalze.

13. ω-(Theophyllin-7-yl)-alkylamino-desoxy-1.4;3.6-dianhydro-hexite der allgemeinen Formel XVI,

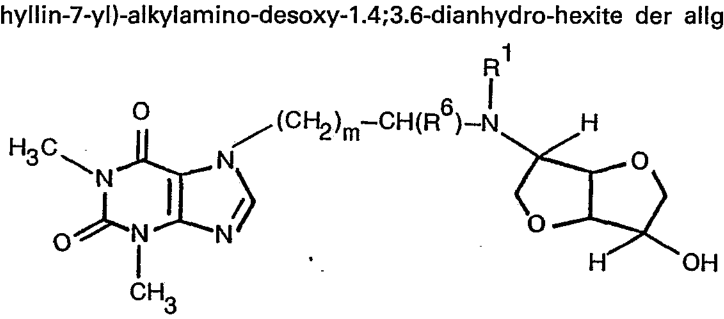

(XVI)

worin $R^1$, $R^6$ und m die in Anspruch 12 genannten Bedeutungen besitzen, sowie deren Säureadditionssalze.

14. 5-Amino-5-desoxy-1.4;3.6-dianhydro-L-idit.

15. 5-Amino-5-desoxy-1.4;3.6-dianhydro-D-glucit.

16. 2-Amino-2-desoxy-1.4;3.6-dianhydro-D-glucit.

17. 2-Amino-2-desoxy-1.4;3.6-dianhydro-D-mannit.

18. 5-Methylamino-5-desoxy-1.4;3.6-dianhydro-L-idit.

19. 5-Äthylamino-5-desoxy-1.4;3.6-dianhydro-L-idit.

20. 5-Adamant-1-yl-amino-5-desoxy-1.4;3.6-dianhydro-L-idit.
21. 5-Dimethylamino-5-desoxy-1.4;3.6-dianhydro-L-idit.
22. 5-Diäthylamino-5-desoxy-1.4;3.6-dianhydro-L-idit.
23. 5-Pyrrolidino-5-desoxy-1.4;3.6-dianhydro-L-idit.
24. 5-Piperidino-5-desoxy-1.4;3.6-dianhydro-L-idit.
25. 5-Morpholino-5-desoxy-1.4;3.6-dianhydro-L-idit.
26. 5-(4-Methylpiperazino)-5-desoxy-1.4;3.6-dianhydro-L-idit.
27. 5-(9-Adenyl)-5-desoxy-1.4;3.6-dianhydro-L-idit.
28. 5-(9-Adenyl)-5-desoxy-1.4;3.6-dianhydro-L-idit-2-methansulfonat.
29. 5-(6-Methylmercaptopurin-9-yl)-5-desoxy-1.4;3.6-dianhydro-L-idit.
30. 5-(6-Methylaminopurin-9-yl)-5-desoxy-1.4;3.6-dianhydro-L-idit.
31. 5-(6-Dimethylaminopurin-9-yl)-5-desoxy-1.4;3.6-dianhydro-L-idit.
32. 5-(6-Äthylaminopurin-9-yl)-5-desoxy-1.4;3.6-dianhydro-L-idit.
33. 5-(6-Pyrrolidinopurin-9-yl)-5-desoxy-1.4;3.6-dianhydro-L-idit.
34. 5-(6-Piperidinopurin-9-yl)-5-desoxy-1.4;3.6-dianhydro-L-idit.
35. 5-(6-Morpholinopurin-9-yl)-5-desoxy-1.4;3.6-dianhydro-L-idit.
36. 5-[6-(4-Methylpiperazino)-purin-9-yl]-5-desoxy-1.4;3.6-dianhydro-L-idit.
37. 5-(6-Benzylaminopurin-9-yl)-5-desoxy-1.4;3.6-dianhydro-L-idit.
38. 5-(6-p-Chlorbenzylaminopurin-9-yl)-5-desoxy-1.4;3.6-dianhydro-L-idit.
39. 5-[6-(3-Phenylpropyl)-aminopurin-9-yl]-5-desoxy-1.4;3.6-dianhydro-L-idit.
40. 5-[6-(3-p-Chlorphenylpropyl)-aminopurin-9-yl]-5-desoxy-1.4;3.6-dianhydro-L-idit.
41. 5-[6-(2-Phenyläthyl)-aminopurin-9-yl]-5-desoxy-1.4;3.6-dianhydro-L-idit.
42. 5-[6-(2-p-Chlorphenyläthyl)-aminopurin-9-yl]-5-desoxy-1.4;3.6-dianhydro-L-idit.
43. 5-[6-(4-Phenylbutyl)-aminopurin-9-yl]-5-desoxy-1.4;3.6-dianhydro-L-idit.
44. 5-[6-(4-p-Chlorphenylbutyl)-aminopurin-9-yl]-5-desoxy-1.4;3.6-dianhydro-L-idit.
45. 2-(9-Adenyl)-2-desoxy-1.4;3.6-dianhydro-D-glucit.
46. 5-(7-Theophyllinyl)-5-desoxy-1.4;3.6-dianhydro-L-idit.
47. 5-Theophyllin-7-yl-5-desoxy-1.4;3.6-dianhydro-L-idit-2-methansulfonat.
48. 5-(2-Theophyllin-7-yl-äthylamino)-5-desoxy-1.4;3.6-dianhydro-L-idit.
49. 5-(3-Theophyllin-7-yl-propylamino)-5-desoxy-1.4;3.6-dianhydro-L-idit.
50. 5-(4-Theophyllin-7-yl-butylamino)-5-desoxy-1.4;3.6-dianhydro-L-idit.
51. 5-(5-Theophyllin-7-yl-pentylamino)-5-desoxy-1.4;3.6-dianhydro-L-idit.
52. 5-(6-Theophyllin-7-yl-hexylamino)-5-desoxy-1.4;3.6-dianhydro-L-idit.
53. 5-(4-Theophyllin-7-yl-2-butylamino)-5-desoxy-1.4;3.6-dianhydro-L-idit.
54. 2-(7-Theophyllinyl)-2-desoxy-1.4;3.6-dianhydro-D-glucit.
55. 2-(7-Theophyllinyl)-2-desoxy-1.4;3.6-dianhydro-D-glucit-5-methansulfonat.
56. 2-(7-Theophyllinyl)-2-desoxy-1.4;3.6-dianhydro-D-glucit-5-p-toluolsulfonat.
57. 2-Dimethylamino-2-desoxy-1.4;3.6-dianhydro-D-glucit.

58. Verfahren zur Herstellung der Verbindungen der allgemeinen Formel I gemäß Anspruch 1, worin $R^3$ ein Wasserstoffatom bedeutet, dadurch gekennzeichnet, daß

(A) L-Isoidid, D-Isosorbid oder D-Isomannid mit einem Sulfonsäurechlorid in den entsprechenden Mono - O - acyl - 1.4;3.6 - dianhydro - hexit übergeführt wird,

(B) welcher durch Ammo nolyse mit wäßriger Ammoniaklösung oder durch Aminolyse mit einem primären oder sekundären Alkylamin mit 1 bis 4 C-Atomen, mit 1-Aminodamantan, einem Alkylisalz des Adenins, 6-Alkylmercaptopurins, 6-Chlorpurins, Theophyllins oder mit einem cyclischen, nicht-aromatischen, gegebenenfalls ein weiteres Heteroatom enthaltenden sekundären Amin das ausgewählt ist aus der Gruppe Pyrrolidin, Piperidin, Morpholin, Methylpiperazin, zum gegebenenfalls entsprechend substituierten Amino - desoxy - 1.4;3.6 - dianhydro - hexit umgesetzt wird,

(C) wonach im Falle des 6-Chlorpurin-9-yl- und des 6-Alkylmercaptopurin-9-yl-desoxy-1.4;3.6-dianhydro-hexits gegebenenfalls anschließend die 6-Chlor- bzw. 6-Alkylmercaptogruppe durch Umsetzung mit einem primären oder sekundären Alkylamin mit 1 bis 4 C-Atomen, einem primären ω-Phenylalkylamin, dessen Alkylrest 1 bis 8 C-Atome aufweist und dessen Phenylrest gegebenenfalls halogensubstituiert ist, oder mit einem cyclischen, nicht-aromatischen, gegebenenfalls ein weiteres Heteroatom enthaltenden sekundären Amin das ausgewählt ist aus der Gruppe Pyrrolidin, Piperidin, Morpholin, Methylpiperazin, entsprechend substituiert wird.

(D) oder wonach im Falle des N-unsubstituierten oder N-monosubstituierten Amino-desoxy-1.4;3.6-dianhydro-hexits gegebenenfalls anschließend die Aminogruppe mit einem ω-Purinyl-1- oder -2-oxoalkan reduktiv kondensiert wird oder mit einem reaktiven ω-Purinyl-alkyl-halogenid, vorzugsweise -chlorid oder -bromid, oder -methansulfonat umgesetzt wird, wobei die Alkylengruppe 2 bis 7 C-Atome aufweist und wobei der Purinylrest den Aden-9-yl- oder Theophyllin-7-yl- oder Theobromin-1-yl-Rest oder einen anderen N,N' - Diniedrigalkyl - xanthin - N'' - yl - Rest, wobei "niedrigalkyl" eine Alkylgruppe mit 1 bis 5 C-Atomen ist, bedeutet.

59. Verfahren zur Herstellung der Verbindungen der allgemeinen Formel V gemäß Anspruch 2, dadurch gekennzeichnet, daß

(A') D-Isosorbid mit einem Überschuß an Methansulfonsäurechlorid oder Toluolsulfonsäurechlorid quantitativ zum entsprechenden Isosorbid-diacylat umgesetzt wird,

(B') welches gemäß Anspruch 58, (B) und gegebenenfalls (C) oder (D) weiter aufgearbeitet und

(C') gegebenenfalls anschließend einer sauren oder alkalischen Hydrolyse unterworfen wird.

60. Verfahren zur Herstellung der Verbindungen der allgemeinen Formel VI gemäß Anspruch 3, worin $R^3$ ein Wasserstoffatom bedeutet, dadurch gekennzeichnet, daß

(A''') D-Isosorbid mit einem Überschuß an Methansulfonsäurechlorid oder Toluolsulfonsäurechlorid quantitativ zum entsprechenden Isosorbid-diacylat umgesetzt wird,

(B''') welches mit Natriumbenzoat selektiv zum entsprechenden 1.4;3.6 - Dianhydro - L - idit - 2 - sulfonat - 5 - benzoat umgesetzt wird,

(C''') wonach letzteres durch Ammonolyse oder Aminolyse gemäß Anspruch 58, (B), wobei gleichzeitig der Sulfonsäurerest hydrolytisch abgespalten wird, zum entsprechenden, gegebenenfalls N-substituierten 5 - Amino - 5 - desoxy - 1.4;3.6 - dianhydro - D - glucit umgesetzt,

(D''') und gegebenenfalls gemäß Anspruch 58, (C) oder (D), weiter aufgearbeitet wird.

61. Verfahren nach Anspruch 60, dadurch gekennzeichnet, daß die Umsetzung mit Natriumbenzoat (B''') in einem wasserfreien, dipolaren, aprotischen Lösungsmittel ggf. unter Inertgasatmosphäre und bei erhöhter Temperatur und erhöhtem Druck durchgeführt wird.

62. Verfahren nach Anspruch 61, dadurch gekennzeichnet, daß Dimethylformamid als aprotisches Lösungsmittel verwendet wird und daß die Umsetzung bei 120—150°C durchgeführt wird.

63. Verfahren nach einem der Ansprüche 58 bis 62, dadurch gekennzeichnet, daß die Ammonolyse oder Aminolyse unter erhöhtem Druck und erhöhter Temperatur durchgeführt wird.

64. Verfahren nach Anspruch 63, dadurch gekennzeichnet, daß die Ammonolyse oder Aminolyse bei 90—180°C durchgeführt wird.

65 Verfahren nach Anspruch 58, dadurch gekennzeichnet, daß die Aminolyse (B) mit einem Alkalisalz des Adenins, 6-Chlorpurins, 6-Alkylmercaptopurins oder Theophyllins unter erhöhter Temperatur in einem dipolaren aprotischen Lösungsmittel ggf. unter Inertgasatmosphäre durchgeführt wird.

66. Verfahren nach Anspruch 65, dadurch gekennzeichnet, daß die Aminolyse bei 90—150°C in Dimethylformamid oder Dimethylsulfoxid durchgeführt wird.

67. Verwendung der Verbindungen gemäß einem der Ansprüche 1 bis 57 als reaktive Zwischenprodukte zur Herstellung von pharmakologisch wirksamen Amino-desoxy-1.4;3.6-dianhydro-hexitnitraten.

**Claims**

1. Aminodesoxy-1.4;3.6-dianhydrohexitol derivatives of the general formula I,

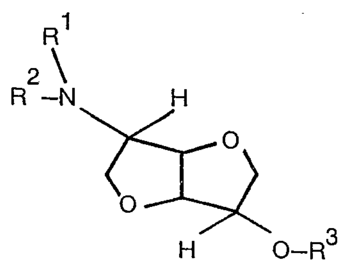

(I)

wherein $R^1$ and $R^2$, in each case independently of one another, signifies a hydrogen atom or a lower alkyl group with 1 to 4 C-atoms or wherein $R^1$ signifies a hydrogen atom and $R^2$ an adament(1)yl radical or wherein $R^1$ and $R^2$, together with the nitrogen atom to which they are attached, (a) signify the residue of a cyclic, non-aromatic secondary amine possibly containing a further hetero atom selected from the group consisting of pyrrolidine, piperidine, morpholine, methylpiperazine, or (b) the aden(9)yl radical, the 6-alkylamino-purin(9)yl or the 6-dialkylamino-purin(9)yl radical with the alkyl groups having 1 to 4 C-atoms, the possibly halogen-substituted 6-(ω-phenylalkyl)-aminopurin(9)yl radical with its alkyl group having 1 to 8 C-atoms, or the 6-pyrrolidinopurin(9)yl-, 6-piperidinopurin(9)yl-, 6-morpholinopurin(9)yl- or 6-methylpiperazinopurin(9)yl radical, or (c) the 6-alkylmercaptopurin(9)yl radical or (d) the theophyllin(7)yl radical or (e) the 6-chloropurin-9-yl radical or wherein $R^1$ signifies a hydrogen atom or a lower alkyl group with 1 to 4 C-atoms and $R^2$ an ω-theophyllin(7)yl-alkyl radical or an ω-theobromin-1-ylalkyl radical or an ω-(N,N'-di-lower alkyl-substituted xanthin-N''-yl)-alkyl radical, whereby "lower alkyl" signifies an alkyl group with 1 to 5 C-atoms, or an ω-adenin-9-ylalkyl radical, whereby the alkyl radical has 2 to 7 C-atoms and can be straight-chained or branched, and wherein $R^3$ signifies a hydrogen atom, a methanesulphonyl or toluenesulphonyl group, as well as their acid-addition salts.

2. 5-Amino-5-desoxy-1.4;3.6-dianhydro-L-iditol derivatives of the general formula V,

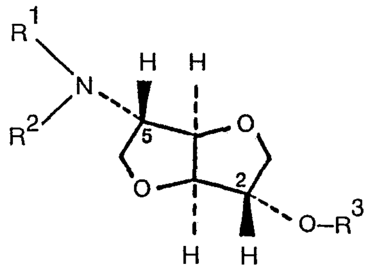

(V)

wherein $R^1$, $R^2$ and $R^3$ possess the meanings given in claim 1, as well as their acid-addition salts.

3. 5-Amino-5-desoxy-1.4;3.6-dianhydro-D-glucitol derivatives of the general formula VI,

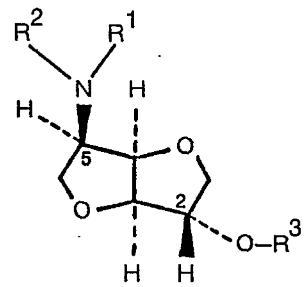

(VI)

wherein $R^1$, $R^2$ and $R^3$ possess the meanings given in claim 1, as well as their acid-addition salts.

4. 2-Amino-2-desoxy-1.4;3.6-dianhydro-D-glucitol derivatives of the general formula VII,

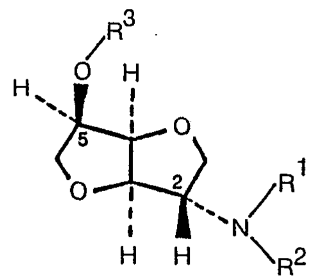

(VII)

wherein $R^1$, $R^2$ and $R^3$ possess the meanings given in claim 1, as well as their acid-addition salts.

5. 2-Amino-2-desoxy-1.4;3.6-dianhydro-D-mannitol derivatives of the general formula VIII,

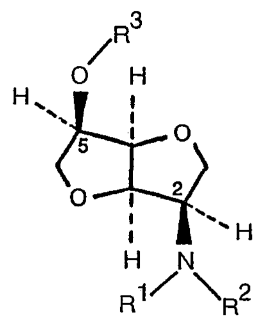

(VIII)

wherein $R^1$, $R^2$ and $R^3$ possess the meanings given in claim 1, as well as their acid-addition salts.

6. (Purin-9-yl)-desoxy-1.4;3.6-dianhydrohexitol derivatives of the general formula IX,

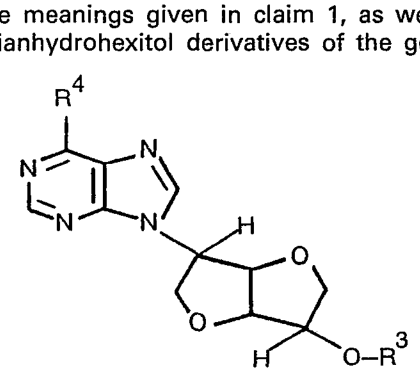

(IX)

wherein $R^3$ signifies a hydrogen atom, a methanesulphonyl or toluenesulphonyl group and $R^4$ an amino, alkylmercapto, alkylamino or dialkylamino group with 1 to 4 C-atoms, an ω-phenylalkylamino group, the alkyl radical of which has 1 to 8 C-atoms and the phenyl radical of which is possibly halogen-substituted or the residue of a cyclic, non-aromatic secondary amine possibly containing a further hetero atom selected from the group consisting of pyrrolidine, piperidine, morpholine, methylpiperazine, or is a chlorine atom, as well as their acid-addition salts.

7. 5-(Purin-9-yl)-5-desoxy-1.4;3.6-dianhydro-L-iditol derivatives of the general formula X,

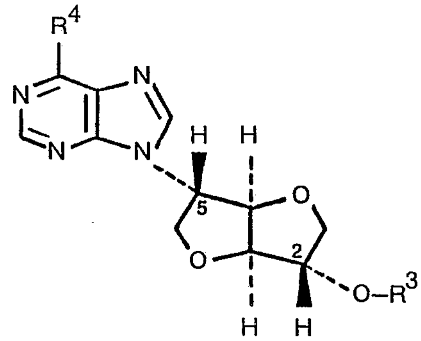

(X)

wherein $R^3$ and $R^4$ possess the meanings given in claim 6, as well as their acid-addition salts.

8. 5-(Purin-9-yl)-5-desoxy-1.4;3.6-dianhydro-D-glucitol derivatives of the general formula XI,

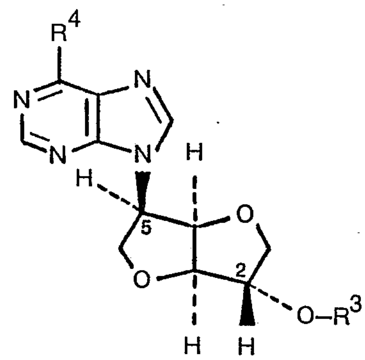

(XI)

wherein $R^3$ and $R^4$ possess the meanings given in claim 6, as well as their acid-addition salts.

9. 2-(Purin-9-yl)-2-desoxy-1.4;3.6-dianhydro-D-glucitol derivatives of the general formula XII,

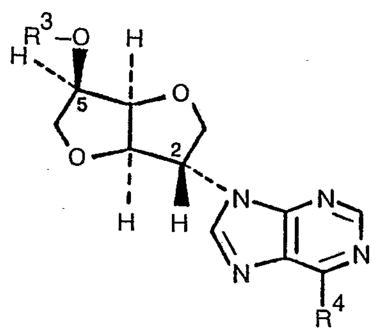

(XII)

wherein $R^3$ and $R^4$ possess the meanings given in claim 6, as well as their acid-addition salts.

10. 2-(Purin-9-yl)-2-desoxy-1.4;3.6-dianhydro-D-mannitol derivatives of the general formula XIII,

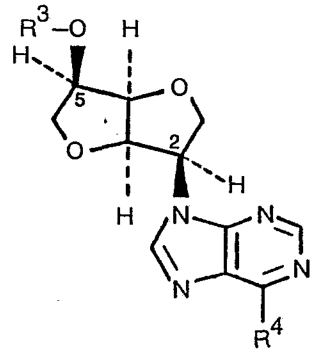

(XIII)

wherein $R^3$ and $R^4$ possess the meanings given in claim 6, as well as their acid-addition salts.

11. (Theophyllin-7-yl)-desoxy-1.4;3.6-dianhydro-hexitol derivatives of the general formula XIV,

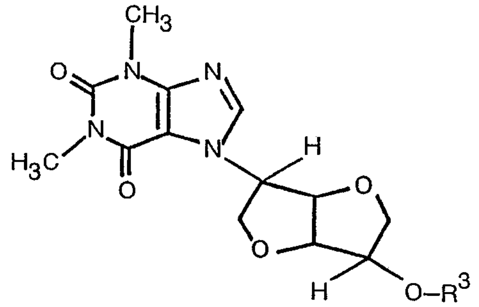

(XIV)

wherein $R^3$ signifies a hydrogen atom, a methanesulphonyl or toluenesulphonyl group, as well as their acid-addition salts.

12. (ω-Purinylalkylamino)-desoxy-1.4;3.6-dianhydro-hexitol derivatives of the general formula XV,

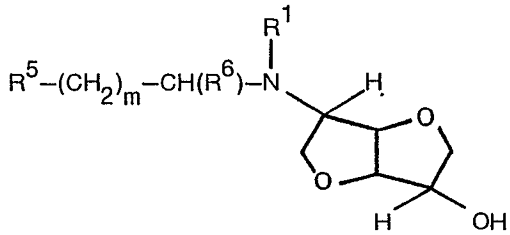

(XV)

wherein $R^1$ signifies a hydrogen atom or a lower alkyl group with 1 to 4 C-atoms, $R^5$ the adenin-9-yl radical, the theophyllin-7-yl radical, the theobromin-1-yl radical or a further N,N'-di-lower alkylxanthin-N''-yl radical, whereby "lower alkyl" means an alkyl group with 1 to 5 C-atoms, $R^6$ signifies a hydrogen atom or a methyl group and *m* a whole number from 1 to 6, as well as their acid-addition salts.

13. ω-(Theophyllin-7-yl)-alkylamino-desoxy-1.4;3.6-dianhydro-hexitols of the general formula XVI,

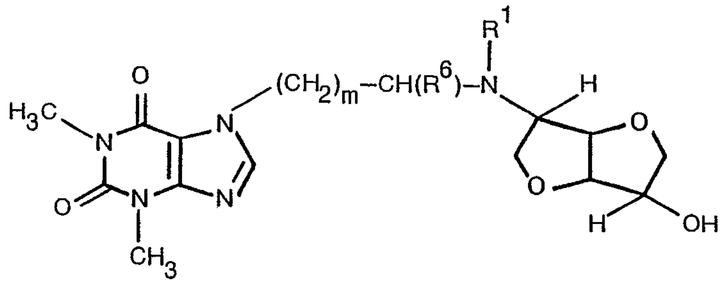

(XVI)

wherein $R^1$, $R^6$ and *m* possess the meanings given in claim 12, as well as their acid-addition salts.

14. 5-Amino-5-desoxy-1.4;3.6-dianhydro-L-iditol.
15. 5-Amino-5-desoxy-1.4;3.6-dianhydro-D-glucitol.
16. 2-Amino-2-desoxy-1.4;3.6-dianhydro-D-glucitol.
17. 2-Amino-2-desoxy-1.4;3.6-dianhydro-D-mannitol.
18. 5-Methylamino-5-desoxy-1.4;3.6-dianhydro-L-iditol.
19. 5-Ethylamino-5-desoxy-1.4;3.6-dianhydro-L-iditol.
20. 5-Adamant-1-ylamino-5-desoxy-1.4;3.6-dianhydro-L-iditol.
21. 5-Dimethylamino-5-desoxy-1.4;3.6-dianhydro-L-iditol.
22. 5-Diethylamino-5-desoxy-1.4;3.6-dianhydro-L-iditol.
23. 5-Pyrrolidino-5-desoxy-1.4;3.6-dianhydro-L-iditol.
24. 5-Piperidino-5-desoxy-1.4;3.6-dianhydro-L-iditol.
25. 5-Morpholino-5-desoxy-1.4;3.6-dianhydro-L-iditol.
26. 5-(4-Methylpiperazino)-5-desoxy-1.4;3.6-dianhydro-L-iditol.
27. 5-(9-Adenyl)-5-desoxy-1.4;3.6-dianhydro-L-iditol.
28. 5-(9-Adenyl)-5-desoxy-1.4;3.6-dianhydro-L-iditol 2-methanesulphonate.
29. 5-(6-Methylmercaptopurin-9-yl)-5-desoxy-1.4;3.6-dianhydro-L-iditol.
30. 5-(6-Methylaminopurin-9-yl)-5-desoxy-1.4;3.6-dianhydro-L-iditol.
31. 5-(6-Dimethylaminopurin-9-yl)-5-desoxy-1.4;3.6-dianhydro-L-iditol.
32. 5-(6-Ethylaminopurin-9-yl)-5-desoxy-1.4;3.6-dianhydro-L-iditol.
33. 5-(6-Pyrrolidinopurin-9-yl)-5-desoxy-1.4;3.6-dianhydro-L-iditol.
34. 5-(6-Piperidinopurin-9-yl)-5-desoxy-1.4;3.6-dianhydro-L-iditol.
35. 5-(6-Morpholinopurin-9-yl)-5-desoxy-1.4;3.6-dianhydro-L-iditol.
36. 5-[6-(4-Methylpiperazino)-purin-9-yl]-5-desoxy-1.4;3.6-dianhydro-L-iditol.

37. 5-(6-Benzylaminopurin-9-yl)-5-desoxy-1.4;3.6-dianhydro-L-iditol.
38. 5-(6-*p*-Chlorobenzylaminopurin-9-yl)-5-desoxy-1.4;3.6-dianhydro-L-iditol.
39. 5-[6-(3-Phenylpropyl)-aminopurin-9-yl]-5-desoxy-1.4;3.6-dianhydro-L-iditol.
40. 5-[6-(3-*p*-Chlorophenylpropyl)-aminopurin-9-yl]-5-desoxy-1.4;3.6-dianhydro-L-iditol.
41. 5-[6-(2-Phenylethyl)-aminopurin-9-yl]-5-desoxy-1.4;3.6-dianhydro-L-iditol.
42. 5-[6-(2-*p*-Chlorophenylethyl)-aminopurin-9-yl]-5-desoxy-1.4;3.6-dianhydro-L-iditol.
43. 5-[6-(4-Phenylbutyl)-aminopurin-9-yl]-5-desoxy-1.4;3.6-dianhydro-L-iditol.
44. 5-[6-(4-*p*-Chlorophenylbutyl)-aminopurin-9-yl]-5-desoxy-1.4;3.6-dianhydro-L-iditol.
45. 2-(9-Adenyl)-2-desoxy-1.4;3.6-dianhydro-D-glucitol.
46. 5-(7-Theophyllinyl)-5-desoxy-1.4;3.6-dianhydro-L-iditol.
47. 5-Theophyllin-7-yl-5-desoxy-1.4;3.6-dianhydro-L-iditol 2-methanesulphonate.
48. 5-(2-Theophyllin-7-ylethylamino)-5-desoxy-1.4;3.6-dianhydro-L-iditol.
49. 5-(3-Theophyllin-7-ylpropylamino)-5-desoxy-1.4;3.6-dianhydro-L-iditol.
50. 5-(4-Theophyllin-7-ylbutylamino)-5-desoxy-1.4;3.6-dianhydro-L-iditol.
51. 5-(5-Theophyllin-7-ylpentylamino)-5-desoxy-1.4;3.6-dianhydro-L-iditol.
52. 5-(6-Theophyllin-7-ylhexylamino)-5-desoxy-1.4;3.6-dianhydro-L-iditol.
53. 5-(4-Theophyllin-7-yl-2-butylamino)-5-desoxy-1.4;3.6-dianhydro-L-iditol.
54. 2-(7-Theophyllinyl)-2-desoxy-1.4;3.6-dianhydro-D-glucitol.
55. 2-(7-Theophyllinyl)-2-desoxy-1.4;3.6-dianhydro-D-glucitol 5-methanesulphonate.
56. 2-(7-Theophyllinyl)-2-desoxy-1.4;3.6-dianhydro-D-glucitol 5-*p*-toluenesulphonate.
57. 2-Dimethylamino-2-desoxy-1.4;3.6-dianhydro-D-glucitol.

58. Process for the preparation of the compounds of the general formula I, according to claim 1, wherein $R^3$ signifies a hydrogen atom, characterised in that

(A) L-isoidide, D-isosorbide or D-isomannide is converted with a sulphonic acid chloride into the corresponding mono - O - acyl - 1.4;3.6 - dianhydrohexitol.

(B) which, by ammonolysis with aqueous ammonia solution or by aminolysis with a primary or secondary alkylamine with 1 to 4 C-atoms, with 1-aminoadamantane, an alkali metal salt of adenine, 6-alkylmercaptopurine, 6-chloropurine, theophylline or with a cyclic, non-aromatic secondary amine possibly containing a further hetero atom selected from the group consisting of pyrrolidine, piperidine, morpholine, methylpiperazine, is reacted to give the possibly correspondingly substituted amino-desoxy-1.4;3.6-dianhydrohexitol,

(C) whereafter, in the case of the 6-chloropurin-9-yl and of the 6-alkylmercaptopurin-9-yl-desoxy-1.4;3.6-dianhydrohexitol, the 6-chloro or 6-alkylmercapto group is possibly subsequently substituted by reaction with a primary or secondary alkylamine with 1 to 4 C-atoms, a primary ω-phenylalkylamine, the alkyl radical of which has 1 to 8 C-atoms and the phenyl radical of which is possibly halogen-substituted, or with a cyclic, non-aromatic secondary amine possibly containing a further hetero atom selected from the group consisting of pyrrolidine, piperidine, morpholine, methylpiperazine,

(D) or whereafter, in the case of the N-unsubstituted or N-monosubstituted aminodesoxy-1.4;3.6-dianhydrohexitol, the amino group is possibly subsequently reductively condensed with an ω-purinyl-1- or -2-oxoalkane or reacted with a reactive ω-purinylalkyl halide, preferably chloride or bromide, or methanesulphonate, whereby the alkylene group has 2 to 7 C-atoms and whereby the purinyl radical signifies the aden-9-yl or theophyllin-7-yl or theobromin-1-yl radical or another N,N′-di-lower alkyl-xanthin-N″-yl radical, whereby "lower alkyl" is an alkyl group with 1 to 5 C-atoms.

59. Process for the preparation of the compounds of the general formula V, according to claim 2, characterised in that

(A′) D-isosorbide is reacted quantitatively with an excess of methanesulphonic acid chloride or toluenesulphonic acid chloride to give the corresponding isosorbide diacylate,

(B′) which is further worked up according to claim 58 (B) and possible (C) or (D) and

(C′) is possibly subsequently subjected to an acidic or alkaline hydrolysis.

60. Process for the preparation of the compounds of the general formula VI, according to claim 3, wherein $R^3$ signifies a hydrogen atom, characterised in that

(A″) D-isosorbide is reacted quantitatively with an excess of methanesulphonic acid chloride or toluenesulphonic acid chloride to give the corresponding isosorbide diacylate,

(B″) which is selectively reacted with sodium benzoate to give the corresponding 1.4;3.6-dianhydro-L-iditol 2-sulphonate-5-benzoate,

(C″) whereafter the latter is reacted by ammonolysis or aminolysis according to claim 58 (B) to give the corresponding possibly N-substituted 5-amino-5-desoxy-1.4;3.6-dianhydro-D-glucitol, whereby the sulphonic acid radical is simultaneously split off hydrolytically,

(D″) and is possibly further worked up according to claim 58 (C) or (D).

61. Process according to claim 60, characterised in that the reaction with sodium benzoate (B″) is carried out in an anhydrous, dipolar, aprotic solvent, possibly under an inert gas atmosphere and at elevated temperature and elevated pressure.

62. Process according to claim 61, characterised in that dimethylformamide is used as an aprotic solvent and in that the reaction is carried out at 120 to 150°C.

63. Process according to one of claims 58 to 62, characterised in that the ammonolysis or aminolysis is carried out under elevated pressure and elevated temperature.

64. Process according to claim 63, characterised in that said ammonolysis or aminolysis is carried out at 90 to 180°C.

63. Process according to claim 58, characterised in that the aminolysis (B) is carried out with an alkali metal salt of adenine, 6-chloropurine, 6-alkylmercaptopurine or theophylline at an elevated temperature in a dipolar, aprotic solvent, possibly under an inert gas atmosphere.

66. Process according to claim 65, characterised in that said aminolysis is carried out at 90 to 150°C in dimethylformamide or dimethyl sulphoxide.

67. Use of the compounds according to any one of claims 1 to 57 as reactive intermediate products for the preparation of pharmacologically-effective amino-desoxy-1.4;3.6-dianhydrohexitol nitrates.

**Revendications**

1. Dérivés de l'amino-désoxy-1,4;3,6-dianhydro-hexite répondant à la formule générale I,

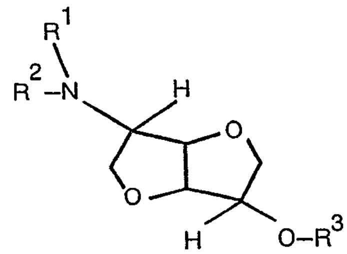

(I)

dans laquelle $R^1$ et $R^2$ désignent chacun indépendamment l'un de l'autre un atome d'hydrogène ou un groupe alkyle inférieur en C1 à C4

ou dans laquelle $R^1$ désigne un atome d'hydrogène et $R^2$ un radical adamant-(1)yle

ou dans laquelle $R^1$ et $R^2$ forment en commun avec l'atome d'azote auquel ils sont liés, a) le radical d'une amine secondaire cyclique, non-aromatique, contenant éventuellement un autre hétéroatome, et choisi dans le groupe de la pyrrolydine, de la pipéridine, de la morpholine, de la méthylpipérazine ou b) le radical aden(9)yle, les radicaux 6 - alkylamino - purin - (9) - yle ou 6 - dialkylamino - purin - (9)yle, les groupes alkyles présentant 1 à 4 atomes de carbone, le radical 6 - (ω - phényl - alkyl) - amino - purin(9)yle éventuellement halogénosubstitué, dont le groupe alkyle présente 1 à 8 atomes de carbone ou le radical 6-pyrrolidinopurin(9)yle, 6-pipéridinopurin(9)yle, 6-morpholinopurin(9)yle ou 6-méthyl-pipérazinopurin(9)yle ou c) le radical 6-alkylmercaptopurin(9)yle ou d) le radical théophylline(7)yle ou e) le radical 6-chloropurin(9)yle

ou dans laquelle $R^1$ désigne un atome d'hydrogène ou un groupe alkyle inférieur en C1 à C4 et $R^2$ un radical ω-théophyllin(7)yle-alkyle ou un radical ω-théobromin-1-yle-alkyle ou un radical ω-(xanthine-N,N'-di(alkyleinférieur)substitué)-N''-yle)alkyle, "alkyle inférieur" désignant und groupe alkyle en C1 à C5, ou un radical ω-adénin-9-yl-alkyle, le radical alkyle présentant à chaque fois 2 à 7 atomes de carbone, et pouvant être linéaires ou ramifiés,

et dans laquelle $R^3$ désigne un atome d'hydrogène, un groupe méthanesulfonyle ou toluènesulfonyle ainsi que leurs sels d'addition aux acides.

Le squelette de base de ces composés consiste en un des 1,4;3,6-dianhydrohexite stéréoisomères, transformables l'un en l'autre par épimérisation, à savoir soit le 1,4;3,6-dianhydro-L-idite (="isoidide") (II),

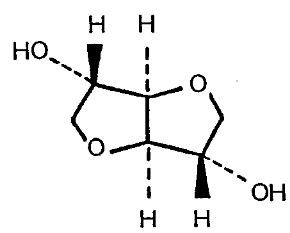

(II)

2. Dérivés du 5-amino-5-désoxy-1,4;3,6-dianhydro-L-idite répondant à la formule générale V;

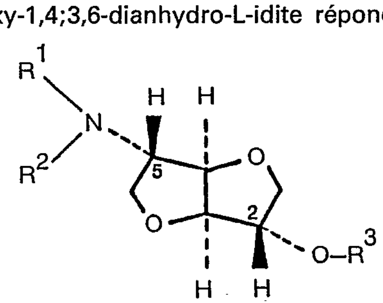

(V)

dans laquelle $R^1$, $R^2$ et $R^3$ possèdent les significations indiquées dans la revendication 1, ainsi que leurs sels d'addition aux acides.

3. Dérivés du 5-amino-5-désoxy-1,4;3,6-dianhydro-D-glucite répondant à la formule générale VI,

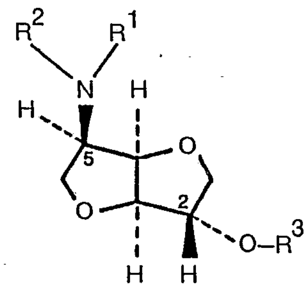

(VI)

dans laquelle $R^1$, $R^2$ et $R^3$ possèdent les significations indiquées dans la revendication 1, ainsi que leurs sels d'addition aux acides.

4. Dérivés du 2-amino-2-désoxy-1,4;3,6-dianhydro-D-glucite répondant à la formule générale VII,

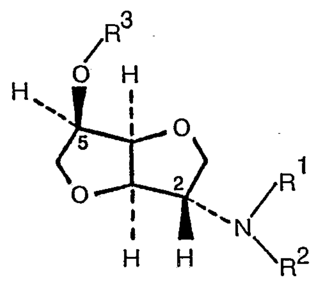

(VII)

dans laquelle $R^1$, $R^2$ et $R^3$ possèdent les significations indiquées dans la revendication 1, ainsi que leurs sels d'addition aux acides.

5. Dérivés du 2-amino-2-désoxy-1,4;3,6-dianhydro-D-mannite répondant à la formule générale VIII,

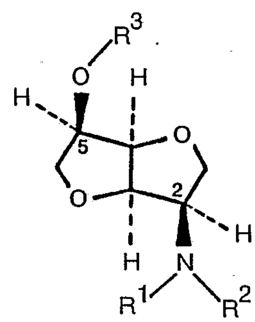

(VIII)

dans laquelle $R^1$, $R^2$ et $R^3$ possèdent les significations indiquées dans la revendication 1, ainsi que leurs sels d'addition aux acides.

6. Dérivés du (purin-9-yl)-désoxy-1,4;3,6-dianhydro-hexite répondant à la formule générale IX,

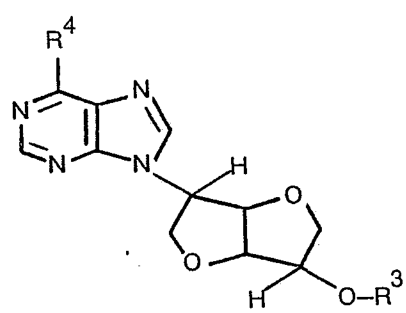

(IX)

dans laquelle $R^3$ désigne un atome d'hydrogène, une groupe méthanesulfonyle ou toluènesulfonyle et $R^4$ désigne un groupe amino, un groupe alkylmercapto, alkylamino ou dialkylamino en C1 à C4, un groupe ω-phénylalkylamino, dont le radical alkyle présente 1 à 8 atomes de carbone et dont le radical est éventuellement halogénosubstitué, ou le radical d'une amine secondaire cyclique, non aromatique, contenant éventuellement un autre hétéroatome, qui est choisi dans le groupe de la pyrrolidine, de la pipéridine, de la morpholine, de la méthylpipérazine, ou un atome de chlore, ainsi que leurs sels d'addition aux acides.

7. Dérivés du 5-(purin-9-yl)-5-désoxy-1,4;3,6-dianhydro-L-idite répondant à la formule générale X,

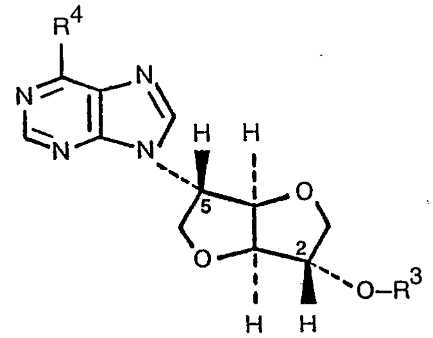

(X)

dans laquelle $R^3$ et $R^4$ possèdent les significations indiquées dans la revendication 6, ainsi que leurs sels d'addition aux acides.

8. Dérivés du (-(purin-9-yl)-5-désoxy-1,4;3,6-dianhydro-D-glucite répondant à la formule générale XI,

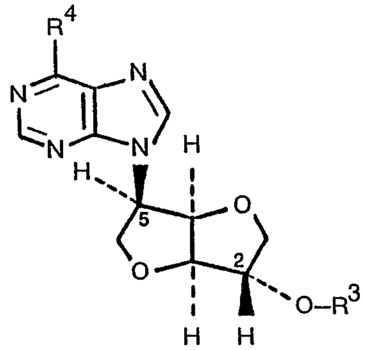

(XI)

dans laquelle $R^3$ et $R^4$ possèdent les significations indiquées dans la revendication 6, ainsi que leurs sels d'addition aux acides.

9. Dérivés du 2-(purin-9-yl)-2-désoxy-1,4;3,6-dianhydro-D-glucite répondant à la formule générale XII,

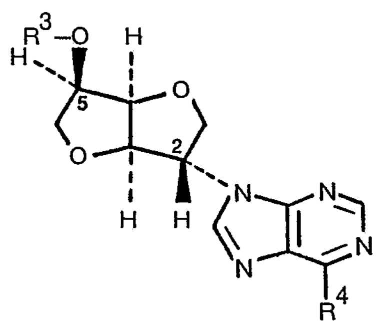

(XII)

dans laquelle $R^3$ et $R^4$ possèdent les significations indiquées dans la revendication 6, ainsi que leurs sels d'addition aux acides.

10. Dérivés du 2-(purin-9-yl)-2-désoxy-1,4;3,6-dianhydro-D-mannite répondant à la formule générale XIII,

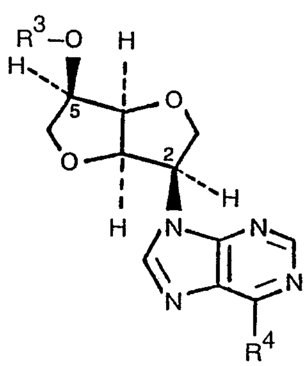

(XIII)

dans laquelle $R^3$ et $R^4$ possèdent les significations indiquées dans la revendication 6, ainsi que leurs sels d'addition aux acides.

11. Dérivés du (théophyllin-7-yl)-désoxy-1,4;3,6-dianhydro-hexite répondant à la formule générale XIV,

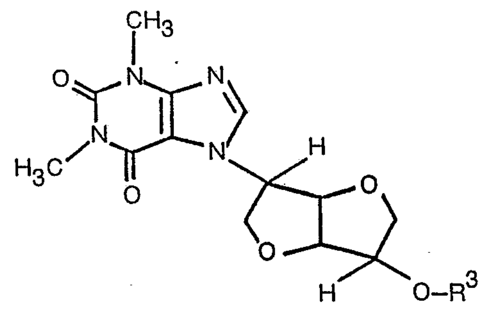

(XIV)

dans laquelle $R^3$ désigne un atome d'hydrogène, un groupe méthanesulfonyle ou toluénesulfonyle, ainsi que leurs sels d'addition aux acides.

12. Dérivés du (ω-purinylalkylamino)-désoxy-1,4;3,6-dianhydro-hexite répondant à la formule générale XV,

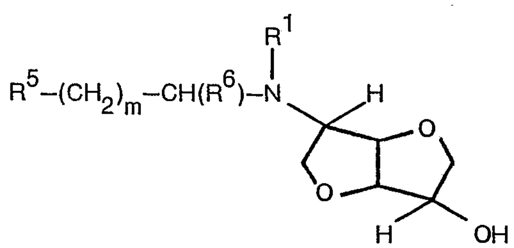

(XV)

dans laquelle $R^1$ désigne un atome d'hydrogène ou un groupe alkyle inférieur en C1 à C4,

$R^5$ désigne le radical adénin-9-yle, le radical théophyllin-7-yle, le radical théobromin-1-yle, ou un autre radical N,N′-di(alkyle inférieur)xanthine-N″-yle, "alkyle inférieur" désignant un groupe alkyle en C1 à C5,

$R^6$ désigne un atome d'hydrogène ou un groupe méthyle et m un nombre entier de 1 à 6, ainsi que leurs sels d'addition aux acides.

13. ω-(théophyllin-7-yl)-alkylamino-désoxy-1,4;3,6-dianhydro-hexite répondant à la formule générale XVI,

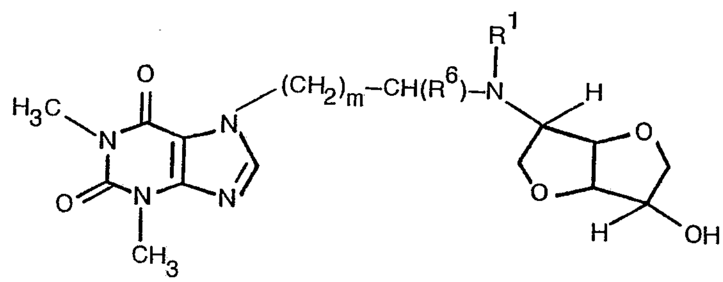

(XVI)

dans laquelle $R^1$, $R^6$ et m possèdent les significations indiquées dans la revendication 12, ainsi que leurs sels d'addition aux acides.

14. 5-amino-5-désoxy-1,4;3,6-dianhydro-L-idite.
15. 5-amino-5-désoxy-1,4;3,6-dianhydro-D-glucite.
16. 2-amino-2-désoxy-1,4;3,6-dianhydro-D-glucite.
17. 2-amino-2-désoxy-1,4;3,6-dianhydro-D-mannite.
18. 5-méthylamino-5-désoxy-1,4;3,6-dianhydro-L-idite.
19. 5-éthylamino-5-désoxy-1,4;3,6-dianhydro-L-idite.
20. 5-adamant-1-yl-amino-5-désoxy-1,4;3,6-dianhydro-L-idite.
21. 5-diméthylamino-5-désoxy-1,4;3,6-dianhydro-L-idite.
22. 5-diéthylamino-5-désoxy-1,4;3,6-dianhydro-L-idite.
23. 5-pyrrolidino-5-désoxy-1,4;3,6-dianhydro-L-idite.
24. 5-pipéridino-5-désoxy-1,4;3,6-dianhydro-L-idite.
25. 5-morpholino-5-désoxy-1,4;3,6-dianhydro-L-idite.
26. 5-(4-méthylpipérazino)-5-désoxy-1,4;3,6-dianhydro-L-idite.
27. 5-(9-adényl)-5-désoxy-1,4;3,6-dianhydro-L-idite.
28. 2-méthanesulfonate de 5-(9-adényl)-5-désoxy-1,4;3,6-dianhydro-L-idite.
29. 5-(6-méthylmercaptopurin-9-yl)-5-désoxy-1,4;3,6-dianhydro-L-idite.
30. 5-(6-méthylaminopurin-9-yl)-5-désoxy-1,4;3,6-dianhydro-L-idite.
31. 5-(6-diméthylaminopurin-9-yl)-5-désoxy-1,4;3,6-dianhydro-L-idite.
32. 5-(6-éthylaminopurin-9-yl)-5-désoxy-1,4;3,6-dianhydro-L-idite.
33. 5-(6-pyrrolidinopurin-9-yl)-5-désoxy-1,4;3,6-dianhydro-L-idite.

34. 5-(6-pipéridinopurin-9-yl)-5-désoxy-1,4;3,6-dianhydro-L-idite.
35. 5-(6-morpholinopurin-9-yl)-5-désoxy-1,4;3,6-dianhydro-L-idite.
36. 5-[6-(4-méthylpipérazino)-purin-9-yl]-5-désoxy-1,4;3,6-dianhydro-L-idite.
37. 5-(6-benzylaminopurin-9-yl)-5-désoxy-1,4;3,6-dianhydro-L-idite.
38. 5-(6-p-chlorobenzylaminopurin-9-yl)-5-désoxy-1,4;3,6-dianhydro-L-idite.
39. 5-[6-(3-phénylpropyl)-aminopurin-9-yl]-5-désoxy-1,4;3,6-dianhydro-L-idite.
40. 5-[6-(3-p-chlorophénylpropyl)-aminopurin-9-yl]-5-désoxy-1,4;3,6-dianhydro-L-idite.
41. 5-[6-(2-phényléthyl)-aminopurin-9-yl]-5-désoxy-1,4;3,6-dianhydro-L-idite.
42. 5-[6-(2-p-chlorophényléthyl)-aminopurin-9-yl]-5-désoxy-1,4;3,6-dianhydro-L-idite.
43. 5-[6-(4-phénylbutyl)-aminopurin-9-yl]-5-désoxy-1,4;3,6-dianhydro-L-idite.
44. 5-[6-(4-p-chlorophénylbutyl)-aminopurin-9-yl]-5-désoxy-1,4;3,6-dianhydro-L-idite.
45. 2-(9-adényl)-2-désoxy-1,4;3,6-dianhydro-D-glucite.
46. 5-(7-théophyllinyl)-5-désoxy-1,4;3,6-dianhydro-L-idite.
47. 2-méthanesulfonate de 5-théophyllin-7-yl-5-désoxy-1,4;3,6-dianhydro-L-idite.
48. 5-(2-théophyllin-7-yl-éthylamino)-5-désoxy-1,4;3,6-dianhydro-L-idite.
49. 5-(3-théophyllin-7-yl-propylamino)-5-désoxy-1,4;3,6-dianhydro-L-idite.
50. 5-(4-théophyllin-7-yl-butylamino)-5-désoxy-1,4;3,6-dianhydro-L-idite.
51. 5-(5-théophyllin-7-yl-pentylamino)-5-désoxy-1,4;3,6-dianhydro-L-idite.
52. 5-(6-théophyllin-7-yl-hexylamino)-5-désoxy-1,4;3,6-dianhydro-L-idite.
53. 5-(4-théophyllin-7-yl-2-butylamino)-5-désoxy-1,4;3,6-dianhydro-L-idite.
54. 2-(7-théophyllinyl)-2-désoxy-1,4;3,6-dianhydro-D-glucite.
55. 5-méthanesulfonate de 2-(7-théophyllinyl)-2-désoxy-1,4;3,6-dianhydro-D-glucite.
56. 5-p-toluènesulfonate de 2-(7-théophyllinyl)-2-désoxy-1,4;3,6-dianhydro-D-glucite.
57. 2-diméthylamino-2-désoxy-1,4;3,6-dianhydro-D-glucite.

58. Procédé de préparation des composés répondant à la formule générale I suivant la revendication 1, dans laquelle $R^3$ désigne un atome d'hydrogène, caractérisé en ce que

(A) On transforme le L-isoidide, le D-isosorbide ou le D-isomannide avec un chlorure de sulfonyle en le 1,4;3,6 - dianhydro - hexite mono - O - acylé correspondant,

(B) que l'on fait réagir par ammonolyse avec une solution aqueuse d'ammoniaque ou par ammynolyse avec une alkylamine primaire ou secondaire en C1 à C4, avec le 1-aminoadamantane, un sel alcalin de l'adénine, une 6-alkylmercaptopurine, la 6-chloropurine, la théophylline, ou avec une amine secondaire cyclique, non aromatique, contenant le cas échéant un autre hétéroatome, qui est choisi dans le groupe de la pyrrolidine, de la pipéridine, de la morpholine, de la méthylpipérazine, pour donner l'amino - désoxy - 1,4;3,6 - dianhydro - hexite convenablement substitué.

(C) après quoi, dans le cas du 6-chloropurin-9-yle et du 6-alkylmercaptopurin-9-yl-désoxy-1,4;3,6-dianhydro-hexite le groupe 6-chloro ou 6-alkylmercapto est ensuite, le cas échéant, substitué convenablement par réaction avec une alkylamine primaire ou secondaire en C1 à C4, une ω-phénylalkylamine primaire, dont le radical alkyle présente 1 à 8 atomes de carbone, et dont le radical phényl est éventuellement halogéno substitué, ou avec une amine secondaire cyclique, non aromatique, contenant le cas échéant un autre hétéroatome, qui est choisi dans le groupe de la pyrrolidine, de la pipéridine, de la morpholine, de la méthylpipérazine,

(D) ou après quoi, dans le cas de l'amino-désoxy-1,4;3,6-dianhydro-hexite N-non substitué ou N-mono substitué, on soumet ensuite le groupe amino à une condensation réductrice avec ω-purinyl-1-ou-2-oxoalkane, ou on le fait réagir avec un halogénure de ω-purynilalkyle réactif, de préférence le chlorure ou le bromure, ou le méthanesulfonate, le groupe alkylène présentant 2 à 16 atomes de carbone, et le radical purinyl désignant le radical aden-9-yle ou théophyllin-7-yle ou thébromin-1-yle ou un autre radical N,N'-di(alkyle inférieur)xanthine-N''-yle, "alkyle inférieur" désignant un groupe alkyle en C1 à C5.

59. Procédé de préparation des composés répondant à la formule générale V, suivant la revendication 2, caractérisé en ce que

(A') on fait réagir le D-isosorbide avec une excès de chlorure de méthanesulfonyle ou de chlorure de toluènesulfonyle quantitativement pour donner le diacylate d'isosorbide correspondant,

(B') qui est traité conformément à la revendication 58, (B) et le cas échéant (C) ou (D), et

(C') le cas échéant soumis ensuite à une hydrolyse acide ou alcaline.

60. Procédé de préparation des composés répondant à la formule générale VI, suivant la revendication 3, dans laquelle $R^3$ désigne un atome d'hydrogène, caractérisé en ce que

(A''') on fait réagir quantitativement le D-isosorbide avec un excès de chlorure de méthylsulfonyle ou de chlorure de toluènesulfonyle pour donner le diacylate d'isosorbide correspondant,

(B''') qui est mis à réagir avec du benzoate de sodium pour donner sélectivement le 2-sulfonate-5-benzoate de 1,4;3,6-dianhydro-L-idite,

(C''') après quoi on coupe hydrolytiquement ce dernier par ammonolyse ou aminolyse suivant la revendication 58, (B), le radical acide sulfonique étant simultanément coupé hydrolytiquement, pour donner le 5-amino-5-désoxy-1,4;3,6-dianhydro-D-glucite correspondant, le cas échéant N-substitué

(D''') et on poursuit le cas échéant le traitement suivant la revendication 58 (C) ou (D).

61. Procédé suivant la revendication 60, caractérisé en ce que la réaction avec le benzoate de sodium

(B''') est effectuée dans un solvant anhydre, dipolaire, aprotique, le cas échéant sous atmosphère de gaz inerte et à chaud et sous pression.

62. Procédé suivant la revendication 61, caractérisé en ce que le diméthylformamide est utilisé comme solvant aprotique et en ce que la réaction est effectuée à 120—150°C.

63. Procédé suivant l'une des revendications 58 à 62, caractérisé en ce que l'ammonolyse ou l'aminolyse est effectuée sous pression et à chaud.

64. Procédé suivant la revendication 63, caractérisé en ce que l'ammonolyse ou l'aminolyse est effectuée à 90—180°C.

65. Procédé suivant la revendication 58, caractérisé en ce que l'aminolyse (B) est effectuée avec un sel alcalin de l'adénine, la 6-chloropurine, une 6-alkylmercaptopurine ou la théophylline à chaud, dans un solvant dipolaire aprotique, le cas échéant sous atmosphère de gaz inerte.

66. Procédé suivant la revendication 65, caractérisé en ce que l'aminolyse est effectuée à 90—150°C dans du diméthylformamide ou du sulfoxyde de diméthyle.

67. Utilisation des composés suivant l'une des revendications 1 à 57 comme intermédiaires réactifs pour la préparation de nitrates d'amino-désoxy-1,4;3,6-dianhydro-hexite pharmacologiquement actifs.